# EUROPÄISCHE PATENTSCHRIFT

(11) **EP 1 346 054 B1**
(45) Veröffentlichungstag und Bekanntmachung des Hinweises auf die Patenterteilung: **26.09.2007**
(21) Anmeldenummer: 01994694.6
(22) Anmeldetag: 21.11.2001
(51) Int. Cl.: C12N 15/56, C12N 9/26, C12N 15/82, C12N 15/63, C12N 5/10, A01H 5/00, C07K 16/40

(54) **NUCLEINSÄUREN, DIE VACUOLÄRE INVERTASEN CODIEREN, PFLANZENZELLEN UND PFLANZEN, DIE DIESE ENTHALTEN SOWIE IHRE VERWENDUNG**
NUCLEIC ACIDS CODING FOR VACUOLAR INVERTASES, VEGETAL CELLS AND PLANTS CONTAINING SAID NUCLEIC ACIDS AND THE USE THEREOF
ACIDES NUCLEIQUES CODANT POUR DES INVERTASES VACUOLAIRES, CELLULES VEGETALES ET PLANTES CONTENANT CES ACIDES NUCLEIQUES, AINSI QUE LEUR UTILISATION

(30) Priorität: 21.12.2000 DE 10063879
(43) Veröffentlichungstag der Anmeldung: 24.09.2003
(73) Patentinhaber: Südzucker Aktiengesellschaft Mannheim/Ochsenfurt, 68165 Mannheim (DE)
(72) Erfinder: GREINER, Steffen, 63073 Offenbach (DE); HARLING, Hinrich, 37085 Göttingen (DE); HARMS, Karsten, 67269 Grünstadt (DE); RAUSCH, Thomas, 69118 Heidelberg (DE); ROSENKRANZ, Heiko, 67067 Ludwigshafen (DE)
(74) Vertreter: Schrell, Andreas
(86) Internationale Anmeldenummer: PCT/EP2001/013523
(87) Internationale Veröffentlichungsnummer: WO 2002/050109

(56) Entgegenhaltungen:
- US-A- 5 658 773
- DATABASE SWISSPROT [Online] 1. August 1992 (1992-08-01) UNGER ET AL.: "BETA-FRUCTOFURANOSIDASE, SOLUBLE ISOENZYME I PRECURSOR (EC 3.2.1.26) SUCROSE-6-PHOSPHATE HYDROLASE) (INVERTASE) (SACCHARASE)" retrieved from EBI, accession no. INVB_DAUCA Database accession no. P80065 XP002200764 & PLANT PHYSIOL, Bd. 104, 1994, Seiten 1351-1357,
- FIEUW S ET AL: "SUGAR TRANSPORT AND SUGAR-METABOLIZING ENZYMES IN SUGAR BEET STORAGE ROOTS (BETA VULGARIS SSP. ALTISSIMA)" JOURNAL OF PLANT PHYSIOLOGY, FISCHER, STUTTGART, DE, Bd. 137, 1990, Seiten 216-223, XP000672996 ISSN: 0176-1617

## Beschreibung

Die vorliegende Erfindung betrifft ein Nucleinsäuremolekül, das eine vacuoläre Invertase codiert, und seine Verwendung zur Modifikation des pflanzlichen Saccharosemetabolismus, insbesondere in der Vacuole der Zelle eines pflanzlichen Speicherorgans. Ferner betrifft die vorliegende Erfindung ein Fragment des Nucleinsäuremoleküls, einen das Nucleinsäuremolekül oder ein Fragment davon enthaltenden Vektor sowie eine Wirtszelle, insbesondere eine Pflanzenzelle, die das Nucleinsäuremolekül oder ein Fragment davon enthält. Die vorliegende Erfindung umfasst ferner eine transgene Pflanze, Verfahren zur Herstellung einer transgenen Pflanze sowie Verfahren zur Modifikation des Saccharosemetabolismus einer Pflanze, insbesondere eines Pflanzenteils.

Während der Lagerung von Zuckerrüben (*Beta vulgaris*), das heisst während des Zeitraums zwischen Ernte und weiterer Verarbeitung, kommt es zu erheblichen Saccharose-Verlusten, die hauptsächlich auf Atmung und Saccharose-Metabolismus zurückzuführen sind. Der Saccharose-Verlust geht mit einer Qualitätsminderung der Zuckerrüben einher, da der Anteil reduzierender Zucker, wie Glucose und Fructose, zunimmt (Burba, M., Zeitschrift für die Zuckerindustrie, 26 (1976), 647-658). Der Saccharoseabbau während der Zuckerrübenlagerung umfasst als ersten metabolischen Schritt eine enzymatische Hydrolyse durch eine vacuoläre Invertase. Für Tomaten ist die Klonierung einer sauren Invertase und eine daraus resultierende Methode zur Erhöhung des Saccharose-Gehalts der Tomaten beschrieben (Bennett et al. US-Patent 5,658,773). Vacuoläre und/oder zellwandgebundene Invertase-Isoformen werden auch bei de novo-Verwundungen von Rübengewebe induziert und synthetisiert (Milling et al., J. Exp. Bot., 44 (1993), 1687-1694). Das heisst, auch bei Verwundungen kommt es zu einer Saccharose-Hydrolyse, um insbesondere Hexosen bereitzustellen, die zur Wundatmung und für den wundaktivierten oxidativen Pentosephosphat (OPP)-Zyklus benötigt werden. Eine genaue Analyse, welche Invertase-Isoformen an der Wundreaktion beteiligt sind, ist allerdings noch nicht durchgeführt worden. Das Verständnis der Regulation und der Rolle der einzelnen Invertase-Isoformen in verwundetem Zuckerrüben-Gewebe ist jedoch von entscheidender Bedeutung, um die Expression der einzelnen Isoformen manipulieren können, insbesondere vor dem Hintergrund der erheblichen Saccharose-Verluste während des Lagerungszeitraums.

Der vorliegenden Erfindung liegt daher das technische Problem zu Grunde, Verfahren und Mittel bereitzustellen, um Pflanzen insbesondere Zuckerrüben, mit einem modifizierten Saccharosemetabolismus herstellen zu können. Die gezielte Veränderung des vacuolären Saccharosemetabolismus in der Pflanzenzelle, insbesondere einer Vacuole dieser Zelle, stellt daher nach wie vor eine wesentliche Grundvoraussetzung für die Bereitstellung von Pflanzen, insbesondere Zuckerrüben, mit verbesserter Lagerstabilität dar.

Erfindungsgemäß wurde das technische Problem gelöst, indem ein pflanzliches Gen, das eine wundinduzierte vacuoläre Invertase (VIwit) codiert, und das codierte Protein identifiziert und charakterisiert wurde. Durch eine gezielte Modulierung der Aktivität, insbesondere Ausschaltung oder Inaktivierung dieser durch Verwundung induzierten vacuolären Invertase können Saccharoseverluste während der Lagerung beziehungsweise nach Verwundung reduziert werden. Erfindungsgemäß wurde erstmalig gezeigt, dass eine pflanzliche vacuoläre, wundinduzierte Invertase durch ein entsprechendes Antisinnkonstrukt in ihrer Aktivität gehemmt wird. Erfindungsgemäß wurde gezeigt, dass der durch Verwundung ausgelöste Saccharoseabbau durch eine einzige, definierte und spezifische vakuoläre InvertaseIsoform, VIwit, verursacht wird. Diese Isoform wurde erfindungsgemäß kloniert. Verschiedene Bereiche der erfindungsgemäß bereitgestellten cDNA-Sequenz können für die Konstruktion von AntisinnKonstrukten, Sinn-Konstrukten, Sinn-Konstrukten für Cosuppression und für Hairpin-Genkonstrukte gemäß Smith et al. (2000 Nature 407, 319-320) zur Ausschaltung der Aktivität dieser wundinduzierten vakuolären Invertase eingesetzt werden. Erfindungsgemäß wird ein, vorzugsweise isoliertes und vollständig gereinigtes, Nucleinsäuremolekül bereitgestellt, ausgewählt aus der Gruppe bestehend aus:
a) einem Nucleinsäuremolekül mit einer in SEQ ID Nr. 1, 2, 3 oder in SEQ ID Nr. 5 dargestellten Nucleotidsequenz oder einem Fragment davon;
b) einem Nucleinsäuremolekül mit einer Sequenz, die ein Protein mit der biologischen Aktivität einer Invertase und mit einer in SEQ ID Nr. 4 oder in SEQ ID Nr. 6 dargestellten Sequenz codiert, oder einem Fragment davon;
c) einem Nucleinsäuremolekül, das zu einem Nucleinsäuremolekül nach a) bis b) komplementär ist, oder einem Fragment davon;
d) einem Nucleinmolekül, das zu einem Nucleinsäuremolekül nach a) bis c) eine Homologie von mindestens 80% aufweist.

Das Nucleinsäuremolekül kann auch ein Nucleinsäuremolekül, das durch Substitution, Addition, Inversion und/oder Deletion einer oder mehrerer Basen eines Nucleinsäuremoleküls nach a) bis d) erhältlich ist, oder ein Nucleinsäuremolekül, das beispielsweise aufgrund der Degeneration des genetischen Codes mit einem Nucleinsäuremolekül nach a) bis d) oder einem Fragment davon hybridisiert, sein.

Ein Nucleinsäuremolekül beziehungsweise eine Nucleinsäure kann eine DNA-Sequenz, zum Beispiel cDNA oder genomische DNA-Sequenz, oder eine RNA-Sequenz, zum Beispiel mRNA-Sequenz sein, insbesondere aus der Zuckerrübe *Beta vulgaris.* Das Nucleinsäuremolekül beziehungsweise die Nucleinsäure liegt vorzugsweise in isolierter und gereinigter Form vor.

Die Erfindung umfasst auch modifizierte Nucleinsäuremoleküle, die beispielsweise durch Substitution, Addition, Inversion und/oder Deletion einer oder mehrerer Basen eines erfindungsgemäßen Nucleinsäuremoleküls, insbesondere innerhalb der codierenden Sequenz einer Nucleinsäure, erhältlich sind, das heißt also auch Nucleinsäuremoleküle, die als Mutanten, Derivate oder funktionellen Äquivalente eines erfindungsgemäßen Nucleinsäuremoleküls bezeichnet werden können. Solche Manipulationen der Sequenzen werden beispielsweise durchgeführt, um die von einer Nucleinsäure codierte Aminosäuresequenz gezielt zu verändern. Nucleinsäuren, die veränderte vacuoläre Invertasen codieren, können unter anderem zur Transformation landwirtschaftlich genutzter Pflanzen verwendet werden, um transgene Pflanzen herzustellen. Gezielte Sequenzveränderungen können auch dem Ziel dienen, innerhalb der Nucleinsäuresequenz geeignete Restriktionsschnittstellen bereitzustellen oder nicht erforderliche Nucleinsäuresequenzen oder Restriktionsschnittstellen zu entfernen. Dabei werden die erfindungsgemäßen Nucleinsäuremoleküle in Plasmiden insertiert und mittels Standardverfahren der Mikrobiologie/Molekularbiologie einer Mutagenese oder einer Sequenzveränderung durch Rekombination unterzogen. Zur Erzeugung von Insertionen, Deletionen oder Substitutionen, wie Transitionen und Transversionen, sind beispielsweise Verfahren zur in vitro-Mutagenese, "primer repair"-Verfahren sowie Restriktions- und/oder Ligationsverfahren geeignet (vgl. Sambrook et al., 1989, Molecular Cloning: A Laboratory Manual, 2. Auflage (1989), Cold Spring Harbor Laboratory Press, NY, USA). Sequenzveränderungen lassen sich auch durch Anlagerung natürlicher oder synthetischer Nucleinsäuresequenzen erreichen. Beispiele für synthetische Nucleinsäuresequenzen sind Adaptoren oder Linker, die u.a. auch zur Verknüpfung von Nucleinsäure-Fragmenten an diese Fragmente angefügt werden.

Die vorliegende Erfindung umfasst auch Nucleinsäuremoleküle, die mit einem der vorstehend beschriebenen Nucleinsäuremoleküle nach a) bis d) hybridisieren. Der im Zusammenhang mit der vorliegenden Erfindung verwendete Ausdruck "Nucleinsäuremolekül, das mit einem Nucleinsäuremolekül nach a) bis d) hybridisiert" bedeutet ein Nucleinsäuremolekül oder eine Nucleinsäure, die unter mäßig stringenten Bedingungen mit einem Nucleinsäuremolekül oder einer Nucleinsäure nach a) bis d) hybridisiert. Beispielsweise kann die Hybridisierung mit einer radioaktiven Gensonde in einer Hybridisierungslösung (25% Formamid; 5 x SSPE; 0,1% SDS; 5 x Denhardt-Lösung; 50 µg/ml Heringsperma-DNA; bezüglich Zusammensetzung der Einzelkomponenten vgl. Sambrook et al., Molecular Cloning: A Laboratory Manual, 2. Auflage (1989), Cold Spring Harbor Laboratory Press, NY, USA) 20 Stunden bei 37°C erfolgen. Anschließend wird unspezifisch gebundene Sonde durch mehrfaches Waschen der Filter in 2 x SSC/0,1% SDS bei 42°C entfernt. Vorzugsweise werden die Filter mit 0,5 x SSC/0,1% SDS, besonders bevorzugt mit 0,1 x SSC/0,1% SDS bei 42°C gewaschen. Diese Nucleinsäuremoleküle weisen in bevorzugter Ausführungsform mindestens 60 %, erfindungsgemäß mindestens 80 %, 85 %, 90 %, 95 %, 98 % und besonders bevorzugt mindestens 99 % Homologie (Identität) auf Nucleinsäureebene zueinander auf.

Verwendbar sind ebenfalls Nucleinsäuremoleküle, die ein Polypeptid oder Protein mit der Aktivität einer vacuolären Invertase codieren, dessen Sequenz mindestens 40%, vorzugsweise mindestens 60%, besonders bevorzugt mindestens 70 %, erfindungsgemäß 80 %, 90 %, 95 % insbesondere 99 % Homologie zu einem Polypeptid oder Protein aufweist, das von einer Nucleinsäure mit einer in SEQ ID Nr. 1, 2, 3 oder in SEQ ID Nr. 5 dargestellten Sequenz codiert wird.

Im Zusammenhang mit der Erfindung bezieht sich der Ausdruck "mindestens 40%, vorzugsweise mindestens 60%, besonders bevorzugt mindestens 70 %, erfindungsgemäß 80 %, 90 %, 95 % insbesondere 99 % Homologie" auf eine Sequenzübereinstimmung auf der Aminosäuresequenz-Ebene, die mit Hilfe bekannter Verfahren, zum Beispiel computergestützter Sequenzvergleiche (Basic local alignment search tool, S.F. Altschul et al., J. Mol. Bio. 215 (1990), 403-410), bestimmt werden kann.

Der dem Fachmann bekannte Ausdruck "Homologie" bezeichnet den Grad der Verwandtschaft zwischen zwei oder mehreren Polypeptid-Molekülen, der durch die Übereinstimmung zwischen den Sequenzen bestimmt wird, wobei Übereinstimmung sowohl eine identische Übereinstimmung als auch einen konservativen Aminosäure-Austausch bedeuten kann. Der Prozentsatz der "Homologie" ergibt sich aus dem Prozentsatz übereinstimmender Bereiche in zwei oder mehr Sequenzen unter Berücksichtigung von Lücken oder anderen Sequenzbesonderheiten.

Der Begriff "konservativer Aminosäure-Austausch" bedeutet den Austausch eines Aminosäurerestes gegen einen anderen Aminosäurerest, wobei dieser Austausch nicht zu einer Änderung der Polarität oder Ladung an der Position der ausgetauschten Aminosäure führt, z. B. der Austausch eines unpolaren Aminosäurerestes gegen einen anderen unpolaren Aminosäurerest. Konservative Aminosäure-Austausche im Zusammenhang mit der Erfindung umfassen beispielsweise: G=A=S, I=V=L=M, D=E, N=Q, K=R, Y=F, S=T G=A=I=V=L=M=Y=F=W=P=S=T.

Die Homologie zwischen miteinander verwandten Polypeptid-Molekülen kann mit Hilfe bekannter Verfahren bestimmt werden. In der Regel werden spezielle Computerprogramme mit den besonderen Anforderungen Rechnung tragenden Algorithmen eingesetzt. Bevorzugte Verfahren zur Bestimmung der Homologie erzeugen zunächst die größte Übereinstimmung zwischen den untersuchten Sequenzen. Computerprogramme zur Bestimmung der Homologie zwischen zwei Sequenzen umfassen, z. B. das GCG-Programmpaket, einschließlich GAP (Devereux, J., et al., Nucleic Acids Research, 12 (12) (1984), 387; Genetics Computer Group University of Wisconsin, Madison (WI)); BLASTP, BLASTN und FASTA (Altschul, S., et al., J. Molec Bio 215 (1990), 403-410). Das BLASTX Programm kann vom National Centre for Biotechnology Information (NCBI) und aus weiteren Quellen bezogen werden (Altschul S., et al., BLAST Handbuch, NCB NLM NIH Bethesda MD 20894; Altschul, S., et al., J. Mol. 215 (1990), 403-410). Auch der bekannte Smith Waterman-Algorithmus kann zur Bestimmung der Homologie verwendet werden.

Bevorzugte Standard-Parameter für den Aminosäuresequenz-Vergleich umfassen beispielsweise: Algorithmus: Needleman und Wunsch, J. Mol. Biol 48 (1970), 443-453; Vergleichsmatrix: BLOSUM 62 von Henikoff und Henikoff, Proc. Natl. Acad. Sci. USA, 89 (1992), 10915-10919; Lücken-Wert (Gap Penalty) : 12 Lückenlängen-Wert (Gap Length Penalty): 4 Schwellenwert der Ähnlichkeit: 0

Auch das GAP-Programm eignet sich zur Verwendung der vorstehend beschriebenen Parameter.

Darüber hinaus lassen sich weitere Algorithmen, Lücken-Öffnungs-Werte, Lückenausdehnungs-Werte und Vergleichsmatrizen einschließlich der im Programm-Handbuch, Wisconsin-Paket, Version 9 (September 1997) beschriebenen verwenden. Die Auswahl der Programme hängt sowohl von dem durchzuführenden Vergleich als auch davon ab, ob der Vergleich zwischen Sequenzpaaren durchgeführt wird, wobei GAP oder Best Fit bevorzugt sind, oder zwischen einer Sequenz und einer umfangreichen Sequenz-Datenbank, wobei FASTA oder BLAST bevorzugt sind.

Die vorliegende Erfindung betrifft ebenfalls ein, vorzugsweise isoliertes und vollständig gereinigtes, Protein, das durch Expression eines erfindungsgemäßen Nucleinsäuremoleküls oder eines Fragments davon in einer Wirtszelle erhältlich ist. Vorzugsweise besitzt das Protein die gleichen vacuolären Invertase-Eigenschaften wie das Protein, das von einem Nucleinsäuremolekül mit einer in SEQ ID Nr. 1, 2, 3 oder in SEQ ID Nr. 5 dargestellten Sequenz codiert wird. Zum Nachweis der Aktivität eines solchen Proteins können die in Beispiel 6 beschriebenen Saccharosespaltungs-Experimente durchgeführt werden.

Im Zusammenhang mit der vorliegenden Erfindung bedeuten die Begriffe "mit der biologischen Aktivität einer Invertase" und "Aktivität einer vacuolären Invertase", dass ein Polypeptid oder Protein unter geeigneten Bedingungen in vitro oder in vivo Saccharose zu Glucose und Fructose spalten kann, beispielsweise in dem in Beispiel 6 beschriebenen Test zur Bestimmung der Invertase-Aktivität.

Verwendbar sind auch isolierte und vollständig oder partiell gereinigte monoclonale oder polyclonale Antikörper oder deren Fragmente, die mit einem erfindungsgemäßen Protein spezifisch und mit einer solchen Affinität reagieren, so dass beispielsweise ein Nachweis dieses Proteins möglich ist.

Die Erfindung betrifft ferner ein Konstrukt, das eine erfindungsgemäße Nucleinsäure und/oder ein Fragment davon unter der Kontrolle von Expressions-Regulationselementen enthält. Im Zusammenhang mit der vorliegenden Erfindung bedeutet ein "Konstrukt", das hier auch als Vektor bezeichnet sein kann, die Kombination einer erfindungsgemäßen Nucleinsäure oder eines Fragments davon mit mindestens einem Nucleinsäure-Zusatzelement, beispielsweise einem Regulationselement. Beispiele für solche Regulationselemente sind konstitutive oder induzierbare Promotoren, wie der *E.coli*-Promotor araBAD (Carra und Schlief, EMBO J., 12 (1993), 35-44) zur Expression in Bakterien, der Hefepromotor PMA1 (Rentsch et al., FEBS Lett., 370 (1995), 264-268) zur Expression in Pilz-Systemen und der virale CaMV35S Promotor (Pietrzak et al., Nucl. Acids Res., 14 (1986), 5857-5868) zur Expression in Pflanzen.

Insbesondere werden erfindungsgemäß speicherorganspezifische Promotoren der Rübe eingesetzt, so dass die erfindungsgemäße Nucleinsäure in einem erwünschten Zielorgan, insbesondere dem Rübenorgan exprimiert wird. Selbstverständlich können auch zeit-, entwicklungs- oder andere gewebespezifische Regulationselemente verwendet werden. Eine besonders bevorzugte Ausführungsform umfasst die Verwendung von wundaktivierten Promotoren der Rübe, so dass die erfindungsgemäße Nucleinsäure erst im Fall einer Verwundung exprimiert wird.

Ferner kann die Nucleinsäure oder das Fragment mit einem Transkriptions-Terminationssignal versehen werden. Derartige Elemente sind bereits beschrieben worden (vgl. zum Beispiel Gielen et al., EMBO J., 8 (1984), 23-29). Die Transkriptionsstart- und -terminationsbereiche können sowohl nativ (homolog) als auch fremdartig (heterolog) zum Wirtsorganismus sein. Die Sequenz der Transkriptionsstart- und -terminationsregionen kann synthetischen oder natürlichen Ursprungs sein oder eine Mischung aus synthetischen und natürlichen Bestandteilen enthalten. In einer besonders bevorzugten Ausführungsform der Erfindung ist das Konstrukt ein Plasmid.

Die Nucleinsäure oder das Fragment kann in dem Konstrukt, insbesondere einem Plasmid, sowohl in Antisinnorientierung als auch in Sinnorientierung zu dem/den regulatorischen Element(en) vorliegen. Liegt die erfindungsgemäße Nucleinsäure oder deren Fragment zum Beispiel in Sinnorientierung zu dem mindestens einen regulatorischen Element, z. B. einem Promotor vor, kann sie, insbesondere nach Transformation und Integration, auch in höherer Kopienzahl, in das Genom, durch Cosuppressionseffekte die endogene vacuoläre Invertaseaktivität hemmen oder reduzieren. Wenn die Nucleinsäure oder das Fragment in dem Konstrukt in Antisinnorientierung zu dem mindestens einen regulatorischen Element vorliegt, kann das Konstrukt beispielsweise in das Genom einer pflanzlichen Wirtszelle eingeführt werden und zur Unterdrückung der Bildung der pflanzeneigenen vacuolären Invertase führen. Unter dem Begriff Genom wird hier sowohl das mitochondriale, plastidäre oder kern-lokalisierte Erbgut in einer Zelle verstanden.

Eine bevorzugte Ausführungsform der vorliegenden Erfindung umfasst daher ein Konstrukt, das eine erfindungsgemäße Nucleinsäure oder ein Fragment davon in Antisinnorientierung zu einem Promotor umfasst, wobei in einer das Konstrukt enthaltenden Wirtszelle die Expression einer vacuolären Invertase gehemmt wird. Dabei umfasst das Nucleinsäure-Fragment vorzugsweise Fragmente mit einer Mindestlänge, die eine Hybridisierung zu endogener Invertase-mRNA oder DNA ermöglichen, beispielsweise mindestens 10 Nucleotide, vorzugsweise mindestens 50 Nucleotide, besonders bevorzugt mindestens 200 Nucleotide. Das Konstrukt, das eine erfindungsgemäße Nucleinsäure oder ein Fragment davon enthält, kann in eine Wirtszelle eingebracht und dort in eine nichttranslatierbare RNA (Antisinn-RNA) transkribiert werden, die durch Bindung an ein endogenes Gen für eine vacuoläre Invertase oder an die daraus transkribierte mRNA die Expression dieses endogenen Gens hemmen kann.

Eine andere bevorzugte Ausführungsform der Erfindung betrifft ein Konstrukt, das eine erfindungsgemäße Nucleinsäure oder ein erfindungsgemäßes Nucleinsäure-Fragment in Sinnorientierung zu einem regulatorischen Element, zum Beispiel einem Promoter, enthält, dem sich eine weitere, gleiche oder andere erfindungsgemäße Nucleinsäure oder ein weiteres, gleiches oder anderes erfindungsgemäßes Nucleinsäure-Fragment in Antisinnorientierung dazu anschließt. Diese Anordnung ermöglicht die Ausbildung einer Doppelstrang-RNA, die den Abbau der endogenen RNA induzieren kann.

In einer weiteren bevorzugten Ausführungsform der Erfindung ist ein Konstrukt vorgesehen, das eine erfindungsgemäße Nucleinsäure in Sinnorientierung zu einem regulatorischen Element, zum Beispiel einem Promotor, enthält. Das Einführen eines solches Konstruktes kann die Transkription eines endogen vorhandenen Gens für die vacuoläre Invertase mittels des Cosuppressionseffektes hemmen oder reduzieren. Dieses Verfahren ist beispielsweise bei Napoli et al. (The Plant Cell (1990) 2, 279 bis 289) oder in den US-Patenten 5,034,323, 5,231,020 und 5,283,184 beschrieben. Eine solche in Sinnorientierung angeordnete Sequenz kann in einer Ausführungsform der Erfindung auch als Fragment mit den für die vorgenannten Antisinnfragmente genannten Mindestlängen eingesetzt werden.

In einer weiteren Ausführungsform ist es erfindungsgemäß vorgesehen, das endogen vorhandene Gen beziehungsweise dessen beiden Allele der vacuolären Invertase durch Mutation dieses Gens beziehungsweise der Allele zu inaktivieren oder in seiner Aktivität zu modifizieren. Dies kann beispielsweise durch Transposon-induzierte Mutagenese oder durch homologe Rekombination unter Verwendung exogen eingeführter erfindungsgemäßer Nucleinsäuren geschehen.

Schließlich ist es erfindungsgemäß auch möglich, mittels katalytischer RNA-Moleküle oder Ribozyme die Expression endogener vakulärer Invertasegene zu hemmen und gemäß dieser Ausführungsform kann es vorgesehen sein, Ribozym-Sequenzen innerhalb erfindungsgemäßer Antisinn-RNA-Sequenzen der vorliegenden Erfindung zu integrieren, um so eine RNAspaltende Aktivität zu erhalten. Die Herstellung und die Verwendung solcher zielspezifischer Ribozyme wird beispielsweise bei Haseloff et al. (Nature (1988), 334, 585 bis 591) beschrieben.

Die vorgenannten Konstrukte zur Modifikation insbesondere Repression, Hemmung oder Unterdrückung endogen vorhandener Aktivität der Invertase oder des Invertasegens müssen nicht notwendigerweise vollständig identische und die vollständige Länge aufweisende erfindungsgemäße Nucleinsäuren enthalten. Vielmehr kann es in vielen Ausführungsformen ausreichend sein, dass die erfindungsgemäß eingesetzten Nucleinsäuren sei es bei der Herstellung von Antisinnkonstrukten oder Cosuppressionskonstrukten nur Fragmente, das heißt Teilsequenzen der erfindungsgemäßen vollständigen Sequenz für die vacuoläre Invertase umfassen. Gleiches gilt für den Identitätsgrad, der durchaus geringer, insbesondere erheblich geringer als eine 100%ige Identität sein kann.

Die Unterdrückung der Expression der wundinduzierten vakuolären Invertaseisoform wird erfindungsgemäß also durch Antisinn-Konstrukte, Sinn-Konstrukte oder Hairpin-Konstrukte (Smith et al. (2000 Nature 407, 319-320) erreicht. Diese Konstrukte werden in bevorzugter Ausführung unter Verwendung der gesamten VIwit-cDNA-Sequenz (SEQ ID Nr. 2), oder ausgewählter Bereiche derselben (SEQ ID Nr. 1 oder 3), kloniert. Jedes Konstrukt kann in einer Ausführungsform unter der Kontrolle des CaMV 35S-Promotors zur Expression gebracht werden, in einer weiteren Ausführungsform unter der Kontrolle eines wundinduzierten Promotors oder in einer weiteren Ausführungsform unter der Kontrolle eines rübenspezifischen Promotors. Für die Antisinn- und Sinn-Konstrukte können Sequenzabschnitte von beispielsweise mindestens 10 Nukleotiden, vorzugsweise mindestens 50 Nukleotiden, besonders bevorzugt mindestens 200 Nukleotiden, eingesetzt werden.

Für die erfindungsgemäße Herstellung von Hairpin-Konstrukten gemäß Smith et al. (2000 Nature 407 319-320) werden vorzugsweise Sequenzabschnitte von beispielsweise mindestens 10 Nukleotiden, vorzugsweise mindestens 50 Nukleotiden, besonders bevorzugt mindestens 200 Nukleotiden der erfindungsgemäßen VIwit-Isoform, 3'-seitig mit entweder einer Spacer-DNA, oder, in einer bevorzugten Ausführungsform, mit einem Intron ligiert. Anschließend wird hieran 3'-seitig die zum ersten Sequenzabschnitt komplementäre Sequenz in Antisinn-Orientierung ligiert. Die in den vorgenannten Hairpin-Konstrukten eingesetzten Intron- oder Spacer-Sequenzen werden hier als Hairpin-Zusatzsequenzen bezeichnet.

In einer anderen bevorzugten Ausführungsform der Erfindung enthält das Plasmid ein Replikationssignal für *E*. *coli* und/oder Hefe und ein Marker-Gen, das eine positive Selektion der mit dem Plasmid transformierten Wirtszellen erlaubt. Wird das Plasmid in eine pflanzliche Wirtszelle eingeführt, so können je nach Einführungsverfahren weitere Sequenzen erforderlich sein, die dem Fachmann bekannt sind. Ist das Plasmid beispielsweise ein Derivat des Ti- oder Ri-Plasmids, so muss die einzuführende Nucleinsäure oder das einzuführende Fragment davon von T-DNA-Sequenzen flankiert sein, die die Integration der Nucleinsäure oder des Fragments davon in das pflanzliche Genom ermöglichen. Die Verwendung von T-DNA zur Transformation von Pflanzenzellen ist intensiv untersucht worden und beispielsweise in EP 120 516; Hoekema, The Binary Plant Vector System, Kapitel V (1985), Offset-drukkerij Kanters B.V. Ablasserdam; Fraley et al., Crit. Rev. Plant Sci., 4 (1985), 1-46, und An et al., EMBO J., 4 (1985), 277-287 beschrieben. Ist die eingeführte Nucleinsäure oder das Fragment davon einmal im Genom integriert, so ist sie/es dort in der Regel stabil und wird auch in der Nachkommenschaft der ursprünglich transformierten Zelle erhalten.

Die im Genom integrierte Sequenz kann ebenfalls einen Selektionsmarker enthalten, der den transformierten Pflanzenzellen Resistenz gegenüber einem Biozid oder einem Antibiotikum, wie Kanamycin, G418, Bleomycin, Hygromycin oder Phosphinotricin, verleiht. Der verwendete Marker gestattet die Selektion transformierter Zellen gegenüber Zellen, in denen die transformierte DNA nicht vorhanden ist.

Erfindungsgemäß kann der Vektor zum Beispiel als Plasmid, Cosmid, Bakteriophage, Virus oder Liposom ausgeführt sein.

Die Erfindung stellt daher auch eine Wirtszelle bereit, die eine erfindungsgemäße Nucleinsäure, insbesondere eine Nucleinsäure mit einer in SEQ ID Nr. 1, 2, 3 oder eine Nucleinsäure mit einer in SEQ ID Nr. 5 dargestellten Sequenz, oder ein Fragment davon oder ein eine erfindungsgemäße Nucleinsäure oder ein Fragment davon umfassendes Konstrukt enthält. Bei der erfindungsgemäßen Wirtszelle kann es sich um ein Bakterium oder eine Hefe-, Insekten-, Säuger- oder Pflanzenzelle handeln.

Ferner betrifft die vorliegende Erfindung eine transgene Pflanze, die in mindestens einer ihrer Zellen eine erfindungsgemäße Nucleinsäure, die ein Protein mit der Aktivität einer, insbesondere vacuolären, Invertase codiert, oder ein Fragment davon oder ein erfindungsgemäßes Konstrukt enthält. Bei transgenen Pflanzen kann es sich um Pflanzen der verschiedensten Arten, Gattungen, Familien, Ordnungen und Klassen handeln, das heißt sowohl monocotyle als auch dicotyle Pflanzen, ebenso wie Algen, Moose, Farne oder Gymnospermae. Transgene Pflanzen können auch Kalli, Pflanzenzellkulturen, sowie Teile, Organe, Gewebe, Ernte- oder Vermehrungsmaterialien dieser Pflanzen umfassen. In der vorliegenden Erfindung handelt es sich bei den transgenen Pflanzen insbesondere um Tabak-, Kartoffel-, Tomaten-, Zuckerrüben-, Zuckerrohr-, Sojabohnen-, Kaffee-, Erbsen-, Bohnen-, Baumwoll-, Reis- oder Maispflanzen.

Im Zusammenhang mit der vorliegenden Erfindung bedeutet der Ausdruck "in mindestens einer ihrer Zellen", dass eine transgene Pflanze mindestens eine Zelle, vorzugsweise jedoch eine Vielzahl von Zellen enthält, die eine oder mehrere stabil integrierte erfindungsgemäße Nucleinsäure oder ein Fragment davon oder ein erfindungsgemäßes Konstrukt enthalten. Die Nucleinsäure oder ein Fragment davon oder ein erfindungsgemäßes Konstrukt kann sowohl im Zellkern als auch im mitochondrialen oder plastidären Genom integriert sein. Vorzugsweise ist die Nucleinsäure, das Fragment oder das Konstrukt an einer Stelle der Zelle integriert, die nicht ihrer/seiner natürlichen Position entspricht, oder in einer Kopienzahl und/oder Orientierung integriert, die nicht der natürlicherweise vorkommenden Kopienzahl und/oder Orientierung entspricht.

Die vorliegende Erfindung betrifft auch ein Verfahren zur Herstellung einer transgenen, vorzugsweise fertilen, Pflanze, das die folgenden Schritte umfasst:
- Einbringen einer erfindungsgemäßen Nucleinsäure, insbesondere einer Nucleinsäure mit einer in SEQ ID Nr. 1, 2, 3 dargestellten Sequenz oder einer Nucleinsäure mit einer in SEQ ID Nr. 5 dargestellten Sequenz, oder eines Fragments davon oder eines eine erfindungsgemäße Nucleinsäure oder ein Fragment davon enthaltenden Konstrukts in eine Pflanzenzelle; und
- Regenerieren einer, vorzugsweise fertilen, Pflanze aus der transformierten Pflanzenzelle, wobei mindestens eine Zelle dieser Pflanze transgen ist, d. h. eine stabil integrierte Nucleinsäure der vorliegenden Erfindung enthält und vorzugsweise exprimiert, d. h. die integrierte Nucleinsäure zu zumindest RNA transcribiert. Vorzugsweise weist die erhaltene Pflanze in mindestens einer ihrer Vacuolen eine reduzierte Invertaseaktivität und damit verbunden erhöhte Saccharosestabilität in der Vacuole auf.

Für das Einbringen einer Nucleinsäure in eine-Pflanzenzelle stehen eine Vielzahl von Verfahren zur Verfügung. Bei den meisten der Verfahren ist es erforderlich, dass die einzuführende Nucleinsäure in einem Konstrukt, wie einem Vektor, vorliegt. Vektoren, beispielsweise Plasmide, Cosmide, Viren, Bakteriophagen, Shuttle-Vektoren und ähnliche sind bekannt. Vektoren umfassen oft Funktionseinheiten, die der Stabilisierung des Vektors in einer Wirtszelle dienen und seine Replikation darin ermöglichen. Außerdem können Vektoren Regulationselemente enthalten, mit denen die Nucleinsäure funktionell verbunden ist und die die Expression der Nucleinsäure ermöglichen.

Neben der Transformation mit Hilfe von Agrobakterien, *beispielsweise Agrobacterium tumefaciens,* stehen noch zahlreiche weitere Verfahren zur Verfügung. Diese Verfahren umfassen die Fusion von Protoplasten, die Mikroinjektion von DNA, die Elektroporation sowie biolistische Verfahren und Virusinfektions-Verfahren. Anders als bei der Transformation mit Hilfe von Agrobakterien, werden bei der Injektion und Elektroporation an sich keine speziellen Anforderungen an den Vektor gestellt. Es können einfache Plasmide wie zum Beispiel pUC-Derivate verwendet werden. Sollen aber aus derartig transformierten Zellen ganze Pflanzen regeneriert werden, ist die Anwesenheit eines selektierbaren Markergens vorteilhaft.

Aus den transformierten Pflanzenzellen können dann in einem geeigneten Medium, das gegebenenfalls Antibiotika oder Biozide zur Selektion enthält, wieder ganze Pflanzen regeneriert werden. Die so erhaltenen Pflanzen können dann auf Anwesenheit der eingeführten DNA getestet werden. Die transformierten Zellen wachsen innerhalb der Pflanzen in der üblichen Weise (siehe auch McCormick et al., 1986, Plant Cell Reports 5, 81-84). Diese Pflanzen können wie üblich angezogen werden und mit Pflanzen, die die gleiche transformierte Erbanlage oder andere Erbanlagen besitzen, gekreuzt werden. Die daraus entstehenden hybriden Individuen weisen die entsprechenden phänotypischen Eigenschaften auf.

Die vorliegende Erfindung umfasst auch ein Verfahren zur Modifikation des Saccharosemetabolismus in Vacuolen einer Pflanze, insbesondere zur Modifikation in Vacuolen einer Zelle eines Speicherorgans der Zuckerrübe, wobei eine erfindungsgemäße Nucleinsäure, insbesondere eine Nucleinsäure mit einer in SEQ ID Nr. 1, 2, 3 dargestellten Sequenz oder eine Nucleinsäure mit einer in SEQ ID Nr. 5 dargestellten Sequenz oder ein Fragment davon in eine Pflanzenzelle und/oder eine Pflanze eingebracht und anschließend eine ganze Pflanze regeneriert wird, wobei in mindestens einer ihrer Zellen eine Expression, d. h. insbesondere Transcription und Translation, der transformierten Nucleinsäure stattfinden kann, so dass eine Pflanze mit, im Vergleich zu nicht transformierten Pflanzen, reduziertem Saccharoseabbau im mindestens einer ihrer Vacuolen erhalten wird.

Zur erfindungsgemäß vorgesehenen Modifikation der Eigenschaften des vacuolären Saccharosemetabolismus einer Pflanze kann sowohl ein Antisinnansatz, ein Ribozymansatz, ein Doppelstrang-RNA-Ansatz aber auch ein Knockout-Ansatz oder Cosuppressionsansatz eingesetzt werden. Beispielsweise betrifft die Erfindung Modifikationen des Saccharosemetabolismus in der Vacuole in einer Pflanze, im Rahmen derer die endogene Aktivität einer Invertase durch Antisinnexpression gehemmt wird. Ebenso kann eine Cosuppression, zum Beispiel durch Einführen mehrerer Genkopien oder von Konstrukten, die die erfindungsgemäße isolierte Nucleinsäuresequenz unter Kontrolle eines starken konstitutiv oder gewebe- und/oder zeitspezifisch exprimierenden Promoters enthalten, bewirkt werden. Derartige die endogen vorhandene Invertase-Aktivität hemmenden Effekte können erfindungsgemäß durch Transformation von Pflänzenzellen mit Antisinnkonstrukten und/oder Integration ein oder mehrerer Sinn-Konstrukte erzielt werden, um einen Cosupressionseffekt zu erhalten. Selbstverständlich können endogen vorhandene vacuoläre Invertasegene mittels der erfindungsgemäßen Nucleinsäuren auch durch z. B. transposon-induzierte Insertion in die endogen vorhandene Nucleinsäure und/oder mittels homologer Rekombination in ihrer Aktivität gehemmt werden.

Die vorliegende Erfindung betrifft also auch die Verwendung der erfindungsgemäßen Nucleinsäuren zur Modifikation des Saccharosemetabolismus von Pflanzen, insbesondere in Vacuolen von deren Speicherorganen.

Die erfindungsgemäßen Nucleinsäuren, insbesondere eine Nucleinsäure mit einer in SEQ ID Nr. 1, 2, 3 dargestellten Sequenz oder eine Nucleinsäure mit einer in SEQ ID Nr. 5 dargestellten Sequenz, können darüber hinaus zur Isolierung homologer Sequenzen aus Bakterien, Pilzen, Pflanzen, Tieren und/oder Menschen verwendet werden. Um nach homologen Sequenzen suchen zu können, müssen zunächst Genbanken angelegt werden, beispielsweise genomische Banken oder oder cDNA-Banken. Mit Hilfe einer Sonde, die z. B. Teile der vorstehend beschriebenen Nucleinsäuren enthält, können dann aus den Genbanken verwandte Sequenzen isoliert werden. Nach Identifizierung und/oder Isolierung der entsprechenden Gene können DNA- und Aminosäuresequenzen bestimmt und die Eigenschaften der von diesen Sequenzen codierten Proteine analysiert werden.

Ferner können die erfindungsgemäßen Nucleinsäuren verwendet werden, um die Expression einer erfindungsgemäßen vacuolären Invertase in pro- und/oder eukaryontischen Zellen zu untersuchen. Werden die vorstehend beschriebenen Nucleinsäuren beispielsweise in prokaryontischen Zellen, wie Bakterien, eingeführt, so wird eine von Bakterien translatierbare RNA-Sequenz gebildet. Diese Bakterienzellen können daher für Untersuchungen der Eigenschaften einer vacuolären Invertase sowie seiner Substrate und Umsetzungsprodukte verwendet werden. Die erfindungsgemäßen Nucleinsäuren können erfindungsgemäß unter der Kontrolle eines regulatorischen Elements in Antisinnorientierung zur Hemmung der Expression einer endogenen Invertase in pro- und/oder eukaryontischen Zellen verwendet werden. Die Herstellung transgener Nutzpflanzen stellt eine weitere Verwendungsmöglichkeit dieser Nucleinsäuren dar.

Das Sequenzprotokoll umfasst:
SEQ ID Nr. 1 zeigt eine 686 Nucleotide umfassende partielle cDNA-Sequenz des VIwit-Gens *aus* Beta *vulgaris.*
SEQ ID Nr. 2 zeigt die 2337 Nucleotide umfassende Sequenz einer vollständigen cDNA-Sequenz des VIwit-Gens aus *Beta vulagaris.*
SEQ ID Nr. 3 zeigt den 1938 Nucleotide umfassenden codierenden Bereich der SEQ ID Nr. 2.
SEQ ID Nr. 4 zeigt die davon abgeleitete 645 Aminosäuren umfassende vollständige Aminosäuresequenz des erfindungsgemäßen VIwit-Proteins.
SEQ ID Nr. 5 zeigt die 7564 Nucleotide umfassende partielle Sequenz des genomischen VIwit-Clons aus *Beta vulgaris.*
SEQ ID Nr. 6 zeigt eine von der SEQ ID Nr. 5 abgeleitete 284 Aminosäuren umfassende partielle Aminosäuresequenz des erfindungsgemäßen VIwit-Proteins (entspricht Positionen 140 bis 423 aus SEQ ID Nr. 4).

Die SEQ ID Nr. 7 und 8 zeigen die für die Amplifikation und Clonierung verwendeten Primer aus *Beta vulgaris.*

Die SEQ ID Nr. 9 und 10 zeigen die Primer aus Beta *vulgaris,* die für die Erzeugung von gegen die N-terminale Protein-Domäne gerichteten Antiseren verwendet wurden.

Weitere vorteilhafte Ausgestaltungen ergeben sich aus den Unteransprüchen.

Die Erfindung wird anhand der folgenden Beispiele und dazugehöriger Figuren näher erläutert.

Die Figuren zeigen:
- Figur 1: ein erfindungsgemäß eingesetztes Antisensekonstrukt mit der erfindungsgemäßen Nucleotidsequenz,
- Figur 2: ein erfindungsgemäß eingesetztes Sense-Konstrukt mit der erfindungsgemäßen Nucleotidsequenz,
- Figur 3: den zeitlichen Verlauf der Induktion von VIwit mRNA in verwundetem Speicherwurzelgewebe von Zuckerrübengewebe, verglichen mit der Induktion von PAL. Für diese Experimente wurden 10 µg Gesamt-RNA pro Bahn geladen und gleichmäßige Beladung durch Ethidiumbromidfärbung und Hybridisierung mit einer 18S-RNA-Probe verifiziert,
- Figur 4: die Induktion von VIwit Protein in verwundetem Speicherwurzelgewebe von Zuckerrübe,
- Figur 5: den zeitlichen Verlauf der Induktion von VIwit-Enzymaktivität in verwundetem Speicherwurzelgewebe von Zuckerrübe. Die Daten sind als Mittelwerte ± Standardabweichung aus drei unterschiedlichen Experimenten angegeben und
- Figur 6: einen RNA-Blot mit VIwit mRNA.

### Beispiel 1

### Pflanzenmaterial

Die diploide *Beta vulgaris* L.-Inzuchtlinie VV-I/ZR 10676 (KWS Saat AG, Einbeck, Deutschland) wurde bis zur Blüte unter Feldbedingungen kultiviert (das heisst 66 Wochen nach Aussaat). Die Rübenkörper wurden im November aus der Erde geerntet und bei 4°C bis April aufbewahrt und dann erneut ausgepflanzt. Für Verwundungs-Experimente wurden 24 Wochen alte Pflanzen geerntet und 3 Monate bei 6°C aufbewahrt. Nachdem das Pflanzenmaterial auf Raumtemperatur gebracht worden war, wurde der Rübenkörper dekapitiert. Aus dem Cortex wurden mit Hilfe eines Korkbohrers Gewebezylinder mit einem Durchmesser von 2 cm entnommen (4 Zylinder pro Rübenkörper). Die Gewebezylinder wurden unmittelbar danach mit einem Satz fixierter Rasierklingen (Dicke: 2 mm) in Scheiben geschnitten. Die Scheiben wurden über unterschiedliche Zeitspannen in feuchter Atmosphäre bei Raumtemperatur inkubiert.

### Beispiel 2

### Clonierung der cDNA-Sequenz und der genomischen DNA-Sequenz der VI-Isoform der Zuckerrübe

Aus verwundetem Rübengewebe wurde nach 3 Tagen RNA isoliert. Die RNA wurde mit Hilfe des RT-PCR-Verfahrens unter Verwendung des Primers VIwit-sense (SEQ ID Nr. 7) mit der Sequenz 5'-ATGGTI(G/C)C(G/T)GA(C/T)C(A/G)(A/T)TGGTA(C/T)GA-3' (I = Inosit) und des Primers VIwit-antisense (SEQ ID Nr. 8) mit der Sequenz 5'-TC(A/G)(G/C)T(A/G)TC-(A/T)G(A/T)(C/T)TC(A/C/T)CCAA(C/T)CCA-3' amplifiziert, wobei eine partielle VIwit-cDNA (SEQ ID Nr. 1) erhalten wurde. Zur Synthese des ersten Stranges wurde dabei reverse MMLV-Transkriptase eingesetzt. Mittels des partiellen Clons wurde eine vollständige cDNA (SEQ ID Nr. 2) für VIwit (Vacuolar Invertase, wound induced in tap roots) cloniert.

Alle PCR-Produkte wurden in den Vektor Bluescript SK+ (Stratagene, Heidelberg, Deutschland) cloniert und in beiden Richtungen sequenziert. Die partielle cDNA wurde als Matrize zur Synthese genspezifischer biotinylierter Sonden gemäß dem Verfahren von Löw und Rausch (in: A laboratory guide to biotinlabelling in biomolecule analysis (Herausgeber: Meier, T. und Fahrenholz, F.), 1996, S. 201-213, Birkhäuser Verlag, Basel) verwendet.

Durch Screenen einer genomischen DNA-Bank, die mit Hilfe des Lambda-ZAP-XhoI-Partial-Fill-In-Vektorkits (Stratagene, Heidelberg, Deutschland) hergestellt worden war (Lehr et al., Plant Mol. Biol., 39 (1999), 463-475), wurden genomische Clone des VIwit-Gens isoliert (SEQ ID Nr. 5).

### Beispiel 3

### RNA-Blot- und Southern-Blot-Analysen

Gesamt-RNA wurde extrahiert, wie von Logemann et al. (Analyt. Biochem., 163 (1987), 16-20) beschrieben. Auf einem 2% Formaldehyd enthaltenden 1,4%-igen Agarose-Gel wurde die Gesamt-RNA aufgetrennt, wobei pro Laufspur 20 µg RNA aufgetragen wurden. Nach Kapillarübertragung auf eine Nylon-Membran (Duralon; Stratagene, Heidelberg, Deutschland) und UV-Vernetzung wurden die Blots mit genspezifischen Sonden hybridisiert.

Aus Zuckerrübenblättern wurde genomische DNA nach dem Verfahren von Murray und Thompson (Nucl. Acids Res., 8 (1980), 4321-4325) isoliert. Nach Spaltung mit den Restriktionsenzymen BglII, EcoRV und XbaI wurden 10 µg RNA auf einem 0,7%-igen Agarose-Gel aufgetrennt und dann auf eine Nylon-Membran übertragen. Die Blots wurden mit den gleichen Sonden wie vorstehend beschrieben hybridisiert.

### Beispiel 4

### Erzeugung von Antiseren

Zum Nachweis des VIwit-Proteins wurde ein Antiserum gegen die N-terminale Proteindomäne erzeugt. Dazu wurde die partielle VIwit-cDNA (SEQ ID Nr. 1) mit zwei Primern, die die Sequenzen 5'-GCCGAATCGAGCTCA-TAAATGGTGTTTGGACAGG-3' (SEQ ID Nr. 9) beziehungsweise 5'-GCCGTTAGAAGCTTAGCCGAGGTATCCAACC-3' (SEQ ID Nr. 10) aufwiesen (wobei diese Sequenzen den Aminosäuresequenzen INGVWTG beziehungsweise GLDTSA entsprechen) amplifiziert. Die Primer enthielten jeweils eine Restriktionsstelle für das Restriktionsenzym SacI beziehungsweise HindIII. Die PCR-Produkte wurden nach Spaltung mit SacI und HindIII gerichtet in den Vektor pQE30 (Qiagen, Hilden, Deutschland) cloniert. Nach Einführung in den E. coli-Stamm M15(pREP4) wurde die N-terminale VIwit-Domäne in diesem Stamm überexprimiert und anschließend mittels Ni-NTA-Affinitätschromatographie aufgereinigt. Das aufgereinigte Protein wurde zur Erzeugung eines polyclonalen Antikörpers in Kaninchen verwendet.

### Beispiel 5

### Immunblot-Analyse

Die Immunblot-Analyse wurde gemäß dem Verfahren von Weil und Rausch (Planta, 193 (1994), 430-437) durchgeführt, wobei allerdings zur Blockierung anstelle von BSA 5% Trockenmilchpulver eingesetzt wurde und die Immunblots unter Verwendung des SuperSignal^{R}-West Dura-Nachweis-Kits (Pierce, Rockford, USA) entwickelt wurden. Bei Kontroll-Blots zeigte sich, dass das gegen VIwit gerichtete Antiserum keine Kreuzreaktivität mit anderen Invertase-Isoformen, beispielsweise Zellwand-Invertasen, zeigte.

### Beispiel 6

### Bestimmung der Aktivität der vacuolären Invertase

Zur Bestimmung der VIwit-Aktivität wurde Gewebe in 30 mM MOPS, pH-Wert 6,0, 250 mM Sorbit, 10 mM MgCl₂, 10 mM KCl und 1 mM PMSF extrahiert. Nach 10-minütiger Zentrifugation (6500 x g, 4°C) wurde der Überstand mit Aceton (Endkonzentration 80%) versetzt, um das Protein zu präzipitieren. Das Proteinpellet wurde in Testpuffer ohne Saccharose resuspendiert. Aliquots wurden auf die Aktivität der löslichen sauren Invertase (VI) in Testpuffer getestet, wobei der Testpuffer 30 mM Saccharose, 20 mM Triethanolamin, 7 mM Citronensäure und 1 mM PMSF, pH-Wert 4,6, enthielt. Freigesetzte Glucose wurde nach dem Verfahren von Weil und Rausch (Planta, 193 (1994), 430-437) in einem gekoppelten Test mit Hexokinase und Glucose-6-phosphat-Dehydrogenase enzymatisch bestimmt.

### Beispiel 7

### Bestimmung der Zucker-Konzentrationen in Geweben

Die Konzentrationen von Hexosen (Glucose und Fructose) im Gewebe des Rübenkörpers wurden nach dem Verfahren von Stitt et al. (Methods in Enzymology, 174 (1989), 518-552) bestimmt.

### Beispiel 8

### Herstellung von Konstrukten zur Pflanzentransformation mit der wundinduzierten vacuolären Invertase (VIwit)

Die VIwit cDNA (2337 bp) liegt "blunt end"-kloniert im Vektor pCR®2.1-TOPO® (Invitrogen) vor. Die VIwit-cDNA (oder ein Fragment derselben) wird entweder aus diesem Vektor mittels Verdau mit Restriktionsendonukleasen herausgeschnitten, oder mittels Polymerasekettenreaktion amplifiziert. Die Klonierung in pBinAR erfolgt dann mittels Standardmethoden der Molekularbiologie (Restriktionsverdau des Vektors, Reinigung von Vektor und Insert, Ligation von Vektor und Insert sowie Transformation des Ligationsansatzes in einen geeigneten Bakterienstamm).

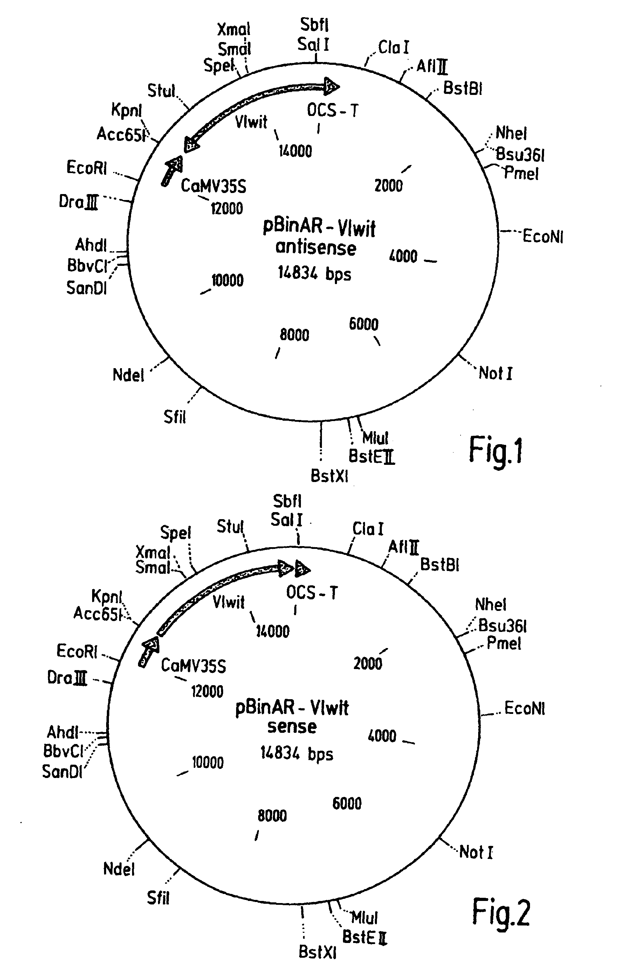

In Figur 1 ist das Konstrukt für VIwit in Antisenseorientierung unter der Kontrolle des CaMV 35S-Promotors in pBinAR (pBin19-Derivat) dargestellt.

In Figur 2 ist ein Konstrukt für VIwit in Senseorientierung im gleichen binären Vektor dargestellt.

### Beispiel 9

### Transformation von Agrobacterium tumefaciens

Der DNA-Transfer in die Agrobakterien erfolgte mittels direkter Transformation nach dem Verfahren von Höfgen und Willmitzer(Nucl. Acids Res., 16 (1988), 9877). Die Plasmid-DNA transformierter Agrobakterien wurde nach dem Verfahren von Birnboim und Doly (Nucl. Acids Res., 7 (1979), 1513-1523) isoliert und nach geeigneter Restriktionsspaltung gelelektrophoretisch analysiert.

### Beispiel 10

### Transformation von Zuckerrüben zur Gewinnung transgener Pflanzen

Etwa einen Monat nach der Keimung von Zuckerrübensamen im Gewächshaus wurden Kalli gemäß dem von Saunders et al. (J. Plant. Physiol., 124 (1986), 473-479) beschriebenen Verfahren induziert. Dabei wurden einer jeden Pflanze junge, drei bis fünf Zentimeter lange Blätter abgenommen, desinfiziert, mit sterilem Wasser dreimal gespült und auf sterilem Filterpapier getrocknet. Jedes Blatt wurde anschließend in Stücke von etwa 0,25 cm² geschnitten und die so erhaltenen Explantate wurden in Petrischalen 30 Tage bei 30°C im Dunklen inkubiert und anschließend in Kulturkammern überführt. Vier bis zehn Wochen nach Kulturbeginn erschienen auf den Blattexplantaten weiße brüchige Kalli. Vier bis sechs Wochen später wurden die Kalli entnommen und in 250 ml-Erlenmeyerkolben in Flüssigmedium kultiviert. Nach etwa zwei bis drei Wochen wurde eine Zellsuspension erhalten.

Nach etwa dreiwöchiger Kultivierung wurden die Zellsuspensionen transformiert. Zu 10 ml Suspensionsmedium wurden 10 ml frisches Medium zugegeben. Die so verdünnte Suspension wurde auf vier Petrischalen verteilt. Aus Stammkulturen von *Agrobacterium* tumefaciens-Stämmen, die mit den erstellten Binärvektoren transformiert worden waren, wurden 50 µl entnommen und in 2 ml LB-Medium, das Rifampicin und Tetracyclin enthielt, kultiviert. Die Kulturen wurden zwei Tage bei 200 U/min und 30°C gerührt. Die Stämme wurden in frisches Medium umgesetzt und unter den vorstehend beschriebenen Bedingungen die Nacht über kultiviert. Die Infektion der Pflanzenzellen erfolgte, indem 50 µl eines Agrobacterium tumefaciens-Stammes einer der die entsprechenden Rübenzellen enthaltenden Petrischalen zugesetzt wurden. Die Rübenzellen und die Bakterien wurden drei Tage lang in einer Kulturkammer in der Dunkelheit kultiviert. Anschließend wurden die Bakterien von den Pflanzenzellen entfernt. Die so gewaschenen Rübenzellen wurden in Petrischalen auf einem Blatt sterilen Whatman-Papier kultiviert und anschließend fünfzehn Tage in der Kulturkammer inkubiert. Drei bis acht Wochen danach erschienen weiße Kalli. Nach einer bestimmten Zeit entwickelten sich aus bestimmten Kalli Sprossen und/oder Embryonen. Sobald die Sprossen mit der Entwicklung von Blättern begonnen hatten, wurden diese auf Medium, das 1 mg/l Naphtalinessigsäure enthielt, eingewurzelt. Nach zwei bis sechs Wochen erschienen Wurzeln. Nach der Entwicklung von Wurzeln wurden die Pflanzen im Gewächshaus in Humuserde akklimatisiert. Nach weiteren drei Monaten hatten sich vollständige Pflanzen entwickelt.

### Beispiel 11:

### VIwit wird in Speicherwurzelgewebe wundinduziert gebildet

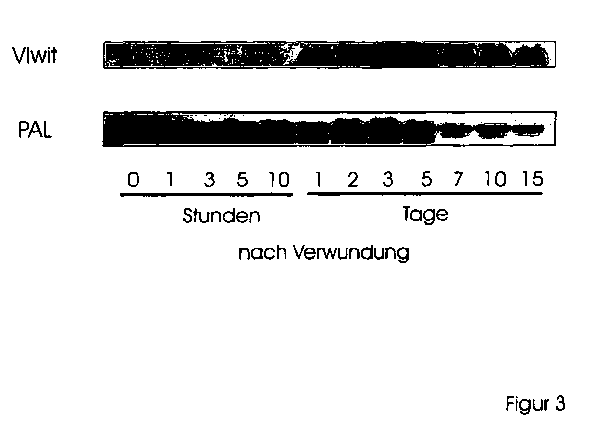

Speicherwurzelgewebe von im Feld gewachsenen Zuckerrüben wurden 24 Wochen nach Auspflanzen geerntet. Zur Herstellung verwundeten Gewebes wurden Gewebezylinder mit einem Durchmesser von 2 cm mit einem Set von Rasierklingen aus dem Cortex herausgeschnitten. Die Schnitte wurden in feuchter Luft bei Raumtemperatur für unterschiedliche Zeiträume von einer Stunde bis 15 Tagen inkubiert. Nach drei Tagen zeigte das Gewebe eine leichte Bräunung, jedoch keine Zeichen bakterieller oder Pilzinfektion. Die Gewebeproben wurden nach den unterschiedlichen Inkubationszeiten gewonnen und auf Transkriptmengen, die Bildung von VIwit und die Aktivität von VIwit untersucht. Der RNA-Blot (Figur 3) mit genspezifischen cDNA-Proben zeigt Wundinduktion von VIwit. VIwit mRNA wird etwa 24 Stunden nach der Verwundung induziert und erreicht ein Maximum nach etwa 5 Tagen. Die mRNA bleibt über den gesamten Zeitraum von 15 Tagen präsent. Es konnten keine Anzeichen von nicht-spezifischem mRNA-Abbau festgestellt werden, was zeigt, dass die zelluläre Integrität nicht negativ beeinflusst wurde. Vergleichsweise wurden die Transkriptmengen von der Phenylalaninammoniumlyase (PAL) bestimmt, einem Gen, das für eine schnelle Induktion nach Verwundung bekannt ist. Die PAL mRNA-Menge stieg drei Stunden nach Verwundung an und erreichte ein Maximum sieben Stunden später. Nach fünf Tagen ging die PAL-mRNA-Menge zurück, wobei jedoch ein Niveau gehalten werden konnte, das höher als das von unverwundetem Gewebe ist.

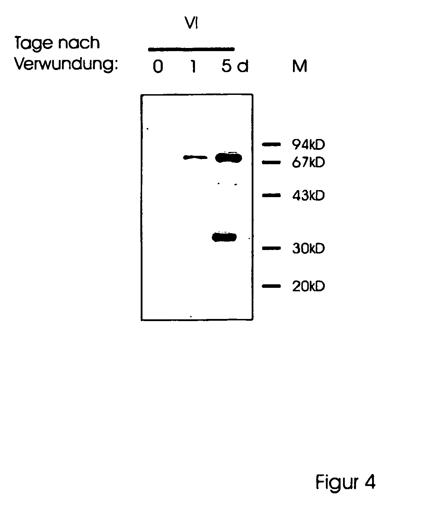

Figur 4 zeigt die Verwendung von Antiseren, die gegen die zentrale Domäne von VIwit gerichtet sind. Die Immunblots reflektieren die Proteinmenge von VIwit. Die Kinetik des Erscheinens von VIwit-Proteinen entspricht der Kinetik der mRNA (siehe Figur 3). Fünf Tage nach Verwundung zeigt die Immunblotanalyse mit dem VIwit-Antiserum ein zusätzliches Polypeptid mit einem Molekulargewicht von etwa 33 kDa, welches aller Wahrscheinlichkeit nach ein Fragment von VIwit darstellt.

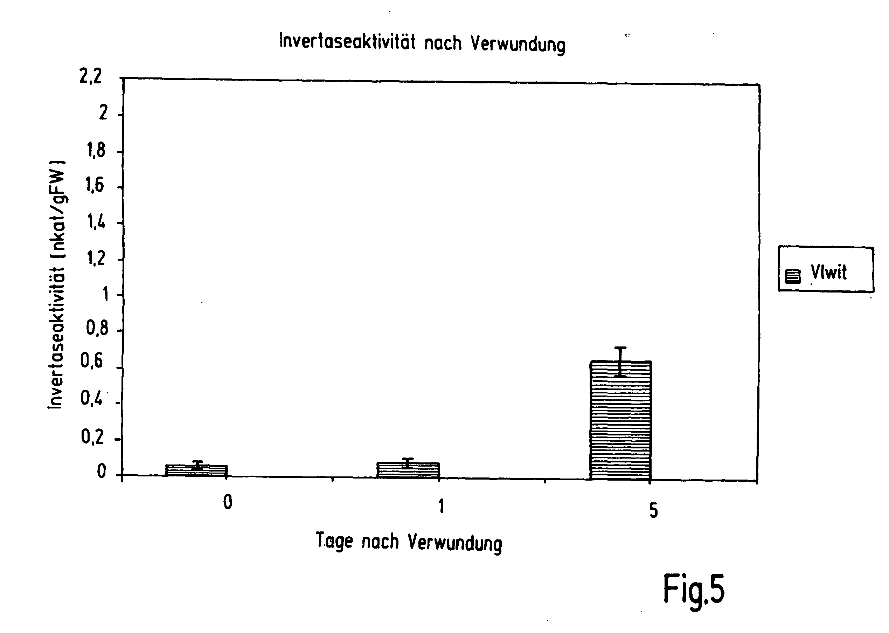

Die aus Figur 5 hervorgehenden saure Invertaseaktivitäten in löslichen Fraktionen aus verwundetem Speicherwurzelgewebe zeigt eine gute Korrelation mit den Daten der Figur 4. Innerhalb der ersten 24 Stunden erhöhte sich die VI-Aktivität um etwa 50%, wobei 4 Tage später eine 7-fache Verstärkung beobachtet werden konnte. Es konnte gezeigt werden, dass VIwit für Aufrechterhalten der erhöhten Hexosemengen während der Wundreaktion verantwortlich ist.

### Beispiel 12

### Expression von VIwit während der Pflanzenentwicklung

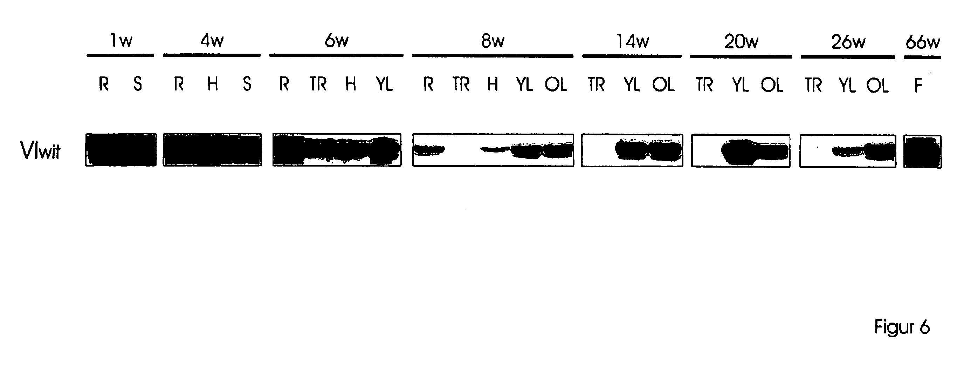

Figur 6 zeigt die Transkriptmengen von VIwit in verschiedenen Pflanzenorganen während der Pflanzenentwicklung (R Wurzel, S gesamter Spross, H Hypocotyl, TR Wurzelspitze, YL junges Blatt (50% der Endgröße), OL voll ausgewachsenes Blatt, F Blüte). Die Pflanzen wurden 1, 4, 6, 8, 14, 20, 26 und 66 Wochen (w) nach Aussäen geerntet. 10 µg Gesamt-RNA wurde pro Bahn geladen und durch gleiche Beladung durch Ethidiumbromidfärbung und Hybridisierung mit einer 18S RNA-Probe verifiziert.

Es konnte gezeigt werden, dass VIwit nicht nur in Wurzeln von Sämlingen, sondern insbesondere auch in Blättern während der gesamten Entwicklung exprimiert wird.

## Patentansprüche

1. Nucleinsäuremolekül, das ein Protein mit der Aktivität einer vacuolären wundinduzierten Invertase codiert, ausgewählt aus der Gruppe bestehend aus:
a) einem Nucleinsäuremolekül mit einer in SEQ ID Nr. 1, 2, 3 oder 5 dargestellten Nucleotidsequenz oder einem Fragment davon;
b) einem Nucleinsäuremolekül mit einer Sequenz, die ein Protein mit einer in SEQ ID Nr. 4 oder 6 dargestellten Sequenz codiert, oder einem Fragment davon;
c) einem Nucleinsäuremolekül, das zu einem Nucleinsäuremolekül nach a) oder b) komplementär ist, oder einem Fragment davon und
d) einem Nucleinsäuremolekül, das zu einem Nucleinsäuremolekül nach a) oder c) eine Homologie von mindestens 80 % aufweist.

2. Nucleinsäuremolekül nach Anspruch 1, **dadurch gekennzeichnet, dass** es eine DNA oder RNA ist, insbesondere aus der Zuckerrübe.

3. Fragment eines Nucleinsäuremoleküls nach Anspruch 1 oder 2, **dadurch gekennzeichnet, dass** es in einer Wirtszelle in Antisinnorientierung zu einem Promoter, oder zusammen mit Ribozym- oder Hairpin-Zusatzsequenzen die Expression einer pflanzlichen vacuolären Invertase hemmen kann.

4. Vektor, enthaltend ein Nucleinsäuremolekül nach Anspruch 1 oder 2 oder ein Nucleinsäure-Fragment nach Anspruch 3 unter der Kontrolle von mindestens einem Expressions-Regulationselement, insbesondere eines Blatt- oder Speicherorgan-spezifischen oder eines wundinduzierten Regulationselements oder in operativer Verknüpfung zusammen mit Ribozym- oder Hairpin-Zusatzsequenzen.

5. Vektor nach Anspruch 4, **dadurch gekennzeichnet, dass** sich das Nucleinsäuremolekül oder das Fragment in Antisinnorientierung zu dem mindestens einen Regulationselement befindet.

6. Vektor nach Anspruch 4 oder 5, **dadurch gekennzeichnet, dass** der Vektor in Form eines Plasmids, Cosmids, Phagen, Liposoms oder Virus vorliegt.

7. Isolierte Wirtszelle, enthaltend ein Nucleinsäuremolekül nach Anspruch 1 oder 2 oder ein Fragment nach Anspruch 3 oder einen Vektor nach einem der Ansprüche 4 bis 6.

8. Wirtszelle nach Anspruch 7, ausgewählt aus Bakterien, Hefezellen, Insektenzellen, Säugerzellen und Pflanzenzellen.

9. Transgene Pflanze, enthaltend in mindestens einer ihrer Zellen ein Nucleinsäuremolekül nach Anspruch 1 oder 2 oder ein Fragment nach Anspruch 3 oder einen Vektor nach einem der Ansprüche 4 bis 6.

10. Transgene Pflanze nach Anspruch 9, **dadurch gekennzeichnet, dass** das Nucleinsäuremolekül, das Fragment oder der Vektor an einer Stelle des Genoms integriert ist, die nicht seiner natürlichen Position entspricht und/oder in einer unnatürlichen Kopienzahl oder Orientierung im Genom integriert ist.

11. Transgene Pflanze nach Anspruch 9 oder 10, wobei die transgene Pflanze Vermehrungs- oder Erntematerial ist.

12. Protein, erhältlich durch Expression eines Nucleinsäuremoleküls nach Anspruch 1 oder 2 in einer Wirtszelle nach Anspruch 7 oder 8.

13. Verfahren zur Herstellung einer transgenen Pflanze, insbesondere nach Anspruch 9 oder 10, umfassend die folgenden Schritte:
- Einbringen eines Nucleinsäuremoleküls nach Anspruch 1 oder 2 oder eines Fragments nach Anspruch 3 in eine Pflanzenzelle; und
- Regenerieren einer Pflanze aus der transformierten Pflanzenzelle.

14. Verfahren zur Modifikation des Saccharosemetabolismus in einer pflanzlichen Vacuole, insbesondere zur Reduktion des Saccharoseabbaus in einer pflanzlichen Vacuole, umfassend das Einbringen eines Nucleinsäuremoleküls nach Anspruch 1 oder 2 oder eines Fragments davon nach Anspruch 3 oder eines das Nucleinsäuremolekül oder das Fragment davon enthaltenden Vektors nach einem der Ansprüche 4 bis 6 in eine Pflanzenzelle und gegebenenfalls das Regenerieren einer ganzen Pflanze daraus, wobei in mindestens einer Vacuole mindestens einer Pflanzenzelle dieser Pflanze ein reduzierter Saccharoseabbau erhalten wird.

15. Verwendung eines Nucleinsäuremoleküls nach Anspruch 1 oder 2 oder eines Fragments davon nach Anspruch 3 zur Isolierung homologer Sequenzen aus Bakterien, Pilzen, Insekten, Pflanzen, Tieren und/oder Menschen.

16. Verwendung eines unter der Kontrolle mindestens eines Regulationselements in Sinn- oder Antisinnorientierung befindlichen Nucleinsäuremoleküls nach Anspruch 1 oder 2 oder eines unter der Kontrolle eines Regulationselements in Sinn- oder Antisinnorientierung befindlichen Fragmentes davon nach Anspruch 3 oder eines mit Ribozym- oder Hairpin-Zusatzsequenzen operativ verknüpften Nucleinsäuremoleküls nach Anspruch 1 oder 2 oder eines Fragmentes davon nach Anspruch 3 zur Reduktion der Expression eines endogenen vacuolären Invertasegens in pro- und/oder eukaryontischen Zellen.

17. Verwendung eines Nucleinsäuremoleküls nach Anspruch 1 oder 2 oder eines Fragments davon nach Anspruch 3 zur Herstellung transgener fertiler Nutzpflanzen mit verändertem vacuolärem Saccharosemetabolismus.

18. Verwendung eines Nucleinsäuremoleküls nach Anspruch 1 oder 2 oder eines Fragments davon nach Anspruch 3 zur Herstellung von Pflanzen, insbesondere Zuckerrüben, mit verbesserter Lagerstabilität, insbesondere ihrer Speicherorgane.

## Claims

1. Nucleic acid molecule encoding a protein with the activity of a vacuolar, wound-induced invertase, selected from the group comprising:
a) a nucleic acid molecule with a nucleotide sequence shown in SEQ ID No. 1, 2, 3 or in SEQ ID No. 5 or a fragment thereof;
b) a nucleic acid molecule with a sequence encoding a protein a sequence shown in SEQ ID No. 4 or in SEQ ID No. 6, or a fragment thereof;
c) a nucleic acid molecule complementary to a nucleic acid molecule according to a) or b), or a fragment thereof; and
d) a nucleic acid molecule that has a homology of at least 80 % to a nucleic acid molecule according to a) or c).

2. Nucleic acid molecule according to claim 1, **characterized in that** it is a DNA or RNA, in particular of the sugar beet.

3. Fragment of a nucleic acid molecule according 1 or 2, **characterized in that** it is able to inhibit the expression of a plant vacuolar invertase in a host cell in antisense orientation to a promoter or together with additional ribozyme or hairpin sequences.

4. Vector, containing a nucleic acid molecule according to claim 1 or 2 or a nucleic acid fragment according to claim 3 under the control of at least one expression regulator element, in particular of a leaf- or storage-organ-specific or wound-induced regulator element or in operative connection together with additional ribozyme or hairpin sequences.

5. Vector according to claim 4, **characterized in that** the nucleic acid molecule or fragment is located in antisense orientation to at least one regulator element.

6. Vector according to claim 4 or 5, **characterized in that** the vector is present in the form of a plasmid, cosmid, phage, liposome, or virus.

7. Isolated host cell, containing a nucleic acid molecule according to claim 1 or 2 or a fragment according to claim 3 or a vector according to one of claims 4 to 6.

8. Host cell according to claim 7, selected from bacteria, yeast cells, insect cells, mammalian cells, and plant cells.

9. Transgenic plant, containing in at least one of its cells a nucleic acid molecule according to claim 1 or 2 or a fragment according to claim 3 or a vector according to one of claims 4 to 6.

10. Transgenic plant according to claim 9, **characterized in that** the nucleic acid molecule, the fragment, or the vector is integrated in a location of the genome that does not correspond to its natural position and/or is integrated in the genome with a number of copies or an orientation not occurring naturally.

11. Transgenic plant according to claim 9 or 10, wherein the transgenic plant is propagation or harvesting material.

12. Protein, obtainable by expression of a nucleic acid molecule according to claim 1 or 2 in a host cell according to claim 7 or 8.

13. Method for producing a transgenic plant, in particular according to claim 9 or 10, comprising the following steps:
- insertion of a nucleic acid molecule according to claim 1 or 2 or a fragment according to claim 3 into a plant cell; and
- regeneration of a plant from the transformed plant cell.

14. Method for modifying the saccharose metabolism in a plant vacuole, in particular for reducing the saccharose break-down in a plant vacuole, comprising the insertion of a nucleic acid molecule according to claim 1 or 2 or a fragment thereof according to claim 3 or a vector containing the nucleic acid molecule or fragment thereof according to one of claims 4 to 6 into a plant cell and potentially the regeneration of an entire plant from it, whereby a reduced saccharose break-down is obtained in at least one vacuole of at least one plant cell of this plant.

15. Use of a nucleic acid molecule according to claim 1 or 2 or a fragment thereof according to claim 3 for isolating homologue sequences from bacteria, fungi, insects, plants, animals, and/or humans.

16. Use of a nucleic acid molecule according to claim 1 or 2 that is under the control of at least one regulator element in sense or antisense orientation, or a fragment thereof according to claim 3 that is under the control of a regulator element in sense or antisense orientation, or a nucleic acid molecule according to claim 1 or 2 that is operatively connected with additional ribozyme or hairpin sequences, or a fragment thereof according to claim 3 for reducing the expression of an endogenous, vacuolar invertase gene in prokaryotic and/or eukaryotic cells.

17. Use of a nucleic acid molecule according to claim 1 or 2 or a fragment thereof according to claim 3 for producing transgenic, fertile crop plants with modified vacuolar saccharose metabolism.

18. Use of a nucleic acid molecule according to claim 1 or 2 or a fragment thereof according to claim 3 for producing plants, especially sugar beets, with improved storage stability, in particular with respect to its storage organs.

## Revendications

1. Molécule d'acide nucléique, laquelle code une protéine présentant l'activité d'une invertase vacuolaire induite par blessure, sélectionnée dans le groupe composé :
a) d'une molécule d'acide nucléique présentant une séquence nucléotidique représentée selon SEQ ID N° 1, 2, 3 ou 5 ou d'un fragment de celle-ci ;
b) d'une molécule d'acide nucléique présentant une séquence qui code une protéine présentant une séquence représentée selon SEQ ID N° 4 ou 6, ou d'un fragment de celle-ci ;
c) d'une molécule d'acide nucléique qui est complémentaire à une molécule d'acide nucléique selon a) ou b), ou d'un fragment de celle-ci ; et
d) d'une molécule d'acide nucléique qui présente une homologie d'au moins 80 % par rapport à une molécule d'acide nucléique selon a) ou c).

2. Molécule d'acide nucléique selon la revendication 1, **caractérisée en ce qu'**elle est un ADN ou ARN, en particulier issu de la betterave sucrière.

3. Fragment d'une molécule d'acide nucléique selon la revendication 1 ou 2, **caractérisé en ce qu'**il peut inhiber l'expression d'une invertase vacuolaire végétale dans une cellule hôte en orientation anti-sens par rapport à un promoteur, ou conjointement avec des séquences supplémentaires ribozymiques ou en épingle à cheveux.

4. Vecteur, comprenant une molécule d'acide nucléique selon la revendication 1 ou 2 ou un fragment d'acide nucléique selon la revendication 3 sous le contrôle d'au moins un élément de régulation d'expression, en particulier un élément de régulation spécifique à une feuille ou un organe de stockage ou un élément de régulation induit par blessure ou en combinaison opérationnelle avec des séquences supplémentaires ribozymiques ou en épingle à cheveux.

5. Vecteur selon la revendication 4, **caractérisé en ce que** la molécule d'acide nucléique ou le fragment se trouve en orientation anti-sens par rapport audit au moins un élément de régulation.

6. Vecteur selon la revendication 4 ou 5, **caractérisé en ce que** le vecteur existe sous forme de plasmide, cosmide, phage, liposome ou virus.

7. Cellule hôte isolée, comprenant une molécule d'acide nucléique selon la revendication 1 ou 2 ou un fragment selon la revendication 3 ou un vecteur selon l'une des revendications 4 à 6.

8. Cellule hôte selon la revendication 7, sélectionnée parmi des bactéries, cellules de levure, cellules d'insectes, cellules de mammifères et cellules végétales.

9. Plante transgénique, comprenant dans au moins une de ses cellules une molécule d'acide nucléique selon la revendication 1 ou 2 ou un fragment selon la revendication 3 ou un vecteur selon l'une des revendications 4 à 6.

10. Plante transgénique selon la revendication 9, **caractérisée en ce que** la molécule d'acide nucléique, le fragment ou le vecteur est intégré(e) à un endroit du génome qui ne correspond pas à sa position naturelle et/ou est intégré(e) dans le génome en un nombre de copies ou selon une orientation non naturel(le).

11. Plante transgénique selon la revendication 9 ou 10, la plante transgénique étant du matériel de multiplication ou de récolte.

12. Protéine, pouvant être obtenue par l'expression d'une molécule d'acide nucléique selon la revendication 1 ou 2 dans une cellule hôte selon la revendication 7 ou 8.

13. Procédé de fabrication d'une plante transgénique, en particulier selon la revendication 9 ou 10, comportant les étapes suivantes :
- introduction d'une molécule d'acide nucléique selon la revendication 1 ou 2 ou d'un fragment selon la revendication 3 dans une cellule végétale ; et
- régénération d'une plante à partir de la cellule végétale transformée.

14. Procédé de modification du métabolisme du saccharose dans une vacuole végétale, en particulier de réduction de la décomposition du saccharose dans une vacuole végétale, comportant l'introduction d'une molécule d'acide nucléique selon la revendication 1 ou 2 ou d'un fragment de celle-ci selon la revendication 3 ou d'un vecteur comprenant la molécule d'acide nucléique ou le fragment de celle-ci selon l'une des revendications 4 à 6 dans une cellule végétale et, le cas échéant, la régénération d'une plante entière à partir de celle-ci (celui-ci), dans lequel une décomposition réduite du saccharose est obtenue dans au moins une vacuole d'au moins une cellule végétale de cette plante.

15. Utilisation d'une molécule d'acide nucléique selon la revendication 1 ou 2 ou d'un fragment de celle-ci selon la revendication 3 pour isoler des séquences homologues issues de bactéries, champignons, insectes, plantes, animaux et/ou êtres humains.

16. Utilisation d'une molécule d'acide nucléique se trouvant sous le contrôle d'au moins un élément de régulation en orientation sens ou anti-sens selon la revendication 1 ou 2 ou d'un fragment de celle-ci se trouvant sous le contrôle d'un élément de régulation en orientation sens ou anti-sens selon la revendication 3 ou d'une molécule d'acide nucléique combinée de manière opérationnelle à des séquences supplémentaires ribozymiques ou en épingle à cheveux selon la revendication 1 ou 2 ou d'un fragment de celle-ci selon la revendication 3 pour réduire l'expression d'un gène d'invertase vacuolaire endogène dans des cellules procaryotes et/ou eucaryotes.

17. Utilisation d'une molécule d'acide nucléique selon la revendication 1 ou 2 ou d'un fragment de celle-ci selon la revendication 3 pour fabriquer des plantes utiles fertiles transgéniques présentant un métabolisme modifié du saccharose vacuolaire.

18. Utilisation d'une molécule d'acide nucléique selon la revendication 1 ou 2 ou d'un fragment de celle-ci selon la revendication 3 pour fabriquer des plantes, en particulier des betteraves sucrières, présentant une meilleure stabilité d'entreposage, en particulier de leurs organes de stockage.


### SEQUENZPROTOKOLL

<110> Südzucker AG Mannheim/Ochsenfurt
<120> Invertasen in Zuckerrüben
<130> 16332
<140>
   <141>
<160> 10
<170> PatentIn Ver. 2.1
<210> 1
   <211> 686
   <212> DNA
   <213> Beta vulgaris
<400> 1
   atgaagcctg atcaatggta tgatataaat ggtgtttgga cagggtccgc tacgatcttg 60
   ccagatggca aaatcatgat ggtctacact ggtgacactg ataaatttgt acaagtccaa 120
   aatttggcat accctgctaa cttatcggat ccattacttc ttgattgggt caaatacccg 180
   ggtaatcctg ttttgacccc accagaggga atcggagcta aagattttcg ggatccgacg 240
   actgcatggg tggggcccga tgggatttgg aggcttatta tcgggtcgaa aacgggtaca 300
   acaggtattt cactagtgta caagaccaag gattttaaga cttatgagct agagagtaac 360
   ctccatgcag ttccgggtac gggtatgtgg gaatgtgtgg atttttaccc ggtgtcgatc 420
   acgggtcaaa atgggttgga tacctcggct tatgggtcgg gtatgaagca cttgttgaag 480
   gctagtttgg atgataataa gcaagatcat tatgcattgg ggacttatga tatgacaact 540
   caaacttgga cacctgataa cccggatatg gatgtgggtc ttgggttgag actggattat 600
   ggtaaatatt atgcgtctaa gacatttttt gaccagaata agcaaaggag gattttgtgg 660
   ggttgggttg gtgagtctga cagtga                                     686
<210> 2
   <211> 2337
   <212> DNA
   <213> Beta vulgaris
<400> 2
   aaatccaacc ttgacaacac ctccactacc ttcccccatt ttctttttta tctatttaga 60
   tctctctcct ctttctctct cttcttatcc tctctctttc tctctccttc ttcaacgacg 120
   aaaaatatgg cttcctactt ctctcccttc gcagacctag aaaacaatga gcccctcctc 180
   cgggcccata atgacccagt ttcagcccaa ccggaacccg aaagcccaac ccaacggccc 240
   attaagggaa tcgcgatact cagcttgtct ttcatgctta tactatcatt aatggcgatc 300
   atatataacg ggcagcctgc ccttctgccc gtatcattgt cgtcggagaa taccgaaacg 360
   gcgtcgtcgg agaaggtggg accagcggtt gtttctagag ggaaaaaaga aggcgtgtcg 420
   gagaagattt caagagtagg agaaagtggt atttcgtttg cgtggactaa tgatatgttg 480
   aggtggcaac gaagtgggtt ccattttcag ccggagaaaa attggatgaa tgatcctaat 540
   ggtccactgt attacaaggg attctatcat ctattttatc agtataaccc tgattcagct 600
   gtttggggga acattacatg gggccatgca atttcaacgg acctgattca ctggaaatat 660
   ctcccaatct caatgaagcc tgatcaatgg tatgatataa atggtgtttg gacagggtcc 720
   gctacgatct tgccagatgg caaaatcatg atggtctaca ctggtgacac tgataaattt 780
   gtacaagtcc aaaatttggc ataccctgct aacttatcgg atccattact tcttgattgg 840
   gtcaaatacc cgggtaatcc tgttttgacc ccaccagagg gaatcggagc taaagatttt 900
   cgggatccga cgactgcatg ggtggggccc gatgggattt ggaggcttat tatcgggtcg 960
   aaaacgggta caacaggtat ttcactagtg tacaagacca aggattttaa gacttatgag 1020
   ctagagagta acctccatgc agttccgggt acgggtatgt gggaatgtgt ggatttttac 1080
   ccggtgtcga tcacgggtca aaatgggttg gatacctcgg cttatgggtc gggtatgaag 1140
   cacttgttga aggctagttt ggatgataat aagcaagatc attatgcatt ggggacttat 1200
   gatatgacaa ctcaaacttg gacacctgat aacccggata tggatgtggg tcttgggttg 1260
   agactggatt atggtaaata ttatgcgtct aagacatttt ttgaccagaa taagcaaagg 1320
   aggattttgt ggggttgggt tggtgagaca gatactgagg ctgatgattt gttgaaggga 1380
   tgggcttctt tgcagacaat tccaagggtt gtgacctatg atgcaaagac cggaaccaac 1440
   atacttcaat ggccagtaaa agaagtggag tccttgagaa cagacagcag agaatatgat 1500
   aatcttttgt tacaaccagg atccatcatt aaccttgaca taacctctgg tgcacagttg 1560
   gatataagtg ctgaatttgt ggtggatcaa gaggcgctga aatcaacagt aggagatgat 1620
   gtgatcaaca attgcagtgc tgcagctgtt aggcaggctt tgggcccgtt tgggcttttg 1680
   gttttggctg atgagtccct ctccgaatta actcctattt acttttacat tgctaaggcc 1740
   tctgatggaa ctgttaaaaa ttggttttgc actgatcaat caaggtcatc aaaggcttct 1800
   agtgttgaca aacaaatata tggaggacca gttcctgttc tcgaaggtga aaagtatagc 1860
   atgcggttac ttgttgatca ttcaataatt gaagcttttg gtcaaggagg gcgtacatgc 1920
   ataacctcta gaatatatcc aacaaaggca atcaacggaa atgccaaggt tttcatcttc 1980
   aataatgcta ctgattcaag tgtcacagcc tctgtcaaaa tctgggaatt ggactcaatt 2040
   aatctttctc cctacatttt ttgatttgat tgttgaattt ttttttattt attgaattgt 2100
   attaatatga tgtactatat atttgtacca attcaaattc taaggggaaa gaaaatgtct 2160
   ttcttttctg ggtggggggt gggggagtgt tgtattgttt gagtcacttc attgtgttaa 2220
   tggttgattt gaggccatga aaataggaat atagatttga tgaataaatg agaattgtaa 2280
   caagggcatg aagagaaatg gaactatgac aaatttaatg gaaaaaaaaa aaaaaaa    2337
<210> 3
   <211> 1938
   <212> DNA
   <213> Beta vulgaris
<400> 3
   atggcttcct acttctctcc cttcgcagac ctagaaaaca atgagcccct cctccgggcc 60
   cataatgacc cagtttcagc ccaaccggaa cccgaaagcc caacccaacg gcccattaag 120
   ggaatcgcga tactcagctt gtctttcatg cttatactat cattaatggc gatcatatat 180
   aacgggcagc ctgcccttct gcccgtatca ttgtcgtcgg agaataccga aacggcgtcg 240
   tcggagaagg tgggaccagc ggttgtttct agagggaaaa aagaaggcgt gtcggagaag 300
   atttcaagag taggagaaag tggtatttcg tttgcgtgga ctaatgatat gttgaggtgg 360
   caacgaagtg ggttccattt tcagccggag aaaaattgga tgaatgatcc taatggtcca 420
   ctgtattaca agggattcta tcatctattt tatcagtata accctgattc agctgtttgg 480
   gggaacatta catggggcca tgcaatttca acggacctga ttcactggaa atatctccca 540
   atctcaatga agcctgatca atggtatgat ataaatggtg tttggacagg gtccgctacg 600
   atcttgccag atggcaaaat catgatggtc tacactggtg acactgataa atttgtacaa 660
   gtccaaaatt tggcataccc tgctaactta tcggatccat tacttcttga ttgggtcaaa 720
   tacccgggta atcctgtttt gaccccacca gagggaatcg gagctaaaga ttttcgggat 780
   ccgacgactg catgggtggg gcccgatggg atttggaggc ttattatcgg gtcgaaaacg 840
   ggtacaacag gtatttcact agtgtacaag accaaggatt ttaagactta tgagctagag 900
   agtaacctcc atgcagttcc gggtacgggt atgtgggaat gtgtggattt ttacccggtg 960
   tcgatcacgg gtcaaaatgg gttggatacc tcggcttatg ggtcgggtat gaagcacttg 1020
   ttgaaggcta gtttggatga taataagcaa gatcattatg cattggggac ttatgatatg 1080
   acaactcaaa cttggacacc tgataacccg gatatggatg tgggtcttgg gttgagactg 1140
   gattatggta aatattatgc gtctaagaca ttttttgacc agaataagca aaggaggatt 1200
   ttgtggggtt gggttggtga gacagatact gaggctgatg atttgttgaa gggatgggct 1260
   tctttgcaga caattccaag ggttgtgacc tatgatgcaa agaccggaac caacatactt 1320
   caatggccag taaaagaagt ggagtccttg agaacagaca gcagagaata tgataatctt 1380
   ttgttacaac caggatccat cattaacctt gacataacct ctggtgcaca gttggatata 1440
   agtgctgaat ttgtggtgga tcaagaggcg ctgaaatcaa cagtaggaga tgatgtgatc 1500
   aacaattgca gtgctgcagc tgttaggcag gctttgggcc cgtttgggct tttggttttg 1560
   gctgatgagt ccctctccga attaactcct atttactttt acattgctaa ggcctctgat 1620
   ggaactgtta aaaattggtt ttgcactgat caatcaaggt catcaaaggc ttctagtgtt 1680
   gacaaacaaa tatatggagg accagttcct gttctcgaag gtgaaaagta tagcatgcgg 1740
   ttacttgttg atcattcaat aattgaagct tttggtcaag gagggcgtac atgcataacc 1800
   tctagaatat atccaacaaa ggcaatcaac ggaaatgcca aggttttcat cttcaataat 1860
   gctactgatt caagtgtcac agcctctgtc aaaatctggg aattggactc aattaatctt 1920
   tctccctaca ttttttga                                               1938
<210> 4
   <211> 645
   <212> PRT
   <213> Beta vulgaris
<400> 4
   Met Ala Ser Tyr Phe Ser Pro Phe Ala Asp Leu Glu Asn Asn Glu Pro
   1               5                   10                  15
   Leu Leu Arg Ala His Asn Asp Pro Val Ser Ala Gln Pro Glu Pro Glu
               20                  25                  30
   Ser Pro Thr Gln Arg Pro Ile Lys Gly Ile Ala Ile Leu Ser Leu Ser
           35                  40                  45
   Phe Met Leu Ile Leu Ser Leu Met Ala Ile Ile Tyr Asn Gly Gln Pro
       50                  55                  60
   Ala Leu Leu Pro Val Ser Leu Ser Ser Glu Asn Thr Glu Thr Ala Ser
   65                  70                  75                  80
   Ser Glu Lys Val Gly Pro Ala Val Val Ser Arg Gly Lys Lys Glu Gly
                   85                  90                  95
   Val Ser Glu Lys Ile Ser Arg Val Gly Glu Ser Gly Ile Ser Phe Ala
               100                 105                 110
   Trp Thr Asn Asp Met Leu Arg Trp Gln Arg Ser Gly Phe His Phe Gln
           115                 120                 125
   Pro Glu Lys Asn Trp Met Asn Asp Pro Asn Gly Pro Leu Tyr Tyr Lys
       130                 135                 140
   Gly Phe Tyr His Leu Phe Tyr Gln Tyr Asn Pro Asp Ser Ala Val Trp
   145                 150                 155                 160
   Gly Asn Ile Thr Trp Gly His Ala Ile Ser Thr Asp Leu Ile His Trp
                   165                 170                 175
   Lys Tyr Leu Pro Ile Ser Met Lys Pro Asp Gln Trp Tyr Asp Ile Asn
               180                 185                 190
   Gly Val Trp Thr Gly Ser Ala Thr Ile Leu Pro Asp Gly Lys Ile Met
           195                 200                 205
   Met Val Tyr Thr Gly Asp Thr Asp Lys Phe Val Gln Val Gln Asn Leu
       210                 215                 220
   Ala Tyr Pro Ala Asn Leu Ser Asp Pro Leu Leu Leu Asp Trp Val Lys
   225                 230                 235                 240
   Tyr Pro Gly Asn Pro Val Leu Thr Pro Pro Glu Gly Ile Gly Ala Lys
                   245                 250                 255
   Asp Phe Arg Asp Pro Thr Thr Ala Trp Val Gly Pro Asp Gly Ile Trp
               260                 265                 270
   Arg Leu Ile Ile Gly Ser Lys Thr Gly Thr Thr Gly Ile Ser Leu Val
           275                 280                 285
   Tyr Lys Thr Lys Asp Phe Lys Thr Tyr Glu Leu Glu Ser Asn Leu His
       290                 295                 300
   Ala Val Pro Gly Thr Gly Met Trp Glu Cys Val Asp Phe Tyr Pro Val
   305                 310                 315                 320
   Ser Ile Thr Gly Gln Asn Gly Leu Asp Thr Ser Ala Tyr Gly Ser Gly
                   325                 330                 335
   Met Lys His Leu Leu Lys Ala Ser Leu Asp Asp Asn Lys Gln Asp His
               340                 345                 350
   Tyr Ala Leu Gly Thr Tyr Asp Met Thr Thr Gln Thr Trp Thr Pro Asp
           355                 360                 365
   Asn Pro Asp Met Asp Val Gly Leu Gly Leu Arg Leu Asp Tyr Gly Lys
       370                 375                 380
   Tyr Tyr Ala Ser Lys Thr Phe Phe Asp Gln Asn Lys Gln Arg Arg Ile
   385 390 395 400
   Leu Trp Gly Trp Val Gly Glu Thr Asp Thr Glu Ala Asp Asp Leu Leu
   405 410 415
   Lys Gly Trp Ala Ser Leu Gln Thr Ile Pro Arg Val Val Thr Tyr Asp
   420 425 430
   Ala Lys Thr Gly Thr Asn Ile Leu Gln Trp Pro Val Lys Glu Val Glu
   435 440 445
   Ser Leu Arg Thr Asp Ser Arg Glu Tyr Asp Asn Leu Leu Leu Gln Pro
   450 455 460
   Gly Ser Ile Ile Asn Leu Asp Ile Thr Ser Gly Ala Gln Leu Asp Ile
   465 470 475 480
   Ser Ala Glu Phe Val Val Asp Gln Glu Ala Leu Lys Ser Thr Val Gly
   485 490 495
   Asp Asp Val Ile Asn Asn Cys Ser Ala Ala Ala Val Arg Gln Ala Leu
   500 505 510
   Gly Pro Phe Gly Leu Leu Val Leu Ala Asp Glu Ser Leu Ser Glu Leu
   515 520 525
   Thr Pro Ile Tyr Phe Tyr Ile Ala Lys Ala Ser Asp Gly Thr Val Lys
   530 535 540
   Asn Trp Phe Cys Thr Asp Gln Ser Arg Ser Ser Lys Ala Ser Ser Val
   545 550 555 560
   Asp Lys Gln Ile Tyr Gly Gly Pro Val Pro Val Leu Glu Gly Glu Lys
   565 570 575
   Tyr Ser Met Arg Leu Leu Val Asp His Ser Ile Ile Glu Ala Phe Gly
   580 585 590
   Gln Gly Gly Arg Thr Cys Ile Thr Ser Arg Ile Tyr Pro Thr Lys Ala
   595 600 605
   Ile Asn Gly Asn Ala Lys Val Phe Ile Phe Asn Asn Ala Thr Asp Ser
   610 615 620
   Ser Val Thr Ala Ser Val Lys Ile Trp Glu Leu Asp Ser Ile Asn Leu
   625 630 635 640
   Ser Pro Tyr Ile Phe
   645
<210> 5
   <211> 7564
   <212> DNA
   <213> Beta vulgaris
<400> 5
   ncnagncatg tangntacca angtgattnt agttagctag ancgtaaaaa cantaaatng 60
   tnggctaatc aagggttgtc agtcaaatgt attgattaat ggagttaata ataaagcatt 120
   agatatgtac ttgatcatat actactatga acacacatgc accccttatt tatttaaccc 180
   aatacaatat acttagaaaa tatctttcag tttaattgtt tttctccttt tcacactaaa 240
   tatatactac tacaagtttg ttttattgca ccatgattca cgagctgccg ccaaacacgg 300
   tcactatctt ccatgcttga cctggcccaa ttattgaagc tctattattt tcataactaa 360
   gctgaattaa tgatttatgt agaaaaatgg acaagatacc taatcatata ctccactaac 420
   ctatcctttc tctaaatagt aagttaaaat ctcatatccc ctaaaaaaaa gttagaatct 480
   tatatctttt gtgaggttag atctataatt ttttttgaca tattataatg aaatgttttt 540
   atttattctc ttattgatcc atgtcaatat ttaggtttgt aatttttggt tttctcgatt 600
   gacatataag tagttagatt gttgaaaaca aatgtttggc aaaactagca tttttagtag 660
   ctgtagctat tgggggtagt tgctagctgt ctactagtag ccgtcaatga tcaatgatta 720
   aattagttgt ttaattagaa gtgtttgaca aaagtagctg ttggaataat aaaaccagag 780
   taaatgatat ttattctaaa atgaaatgaa ctaaaacaat ttcatatgtt cgttcaatag 840
   ttaaattcaa tatttatttt ttcaaagaag ttacacaaaa gagaggtata caaattttat 900
   agctgctcaa aaaaatgtat aaatctaaga aaatattcag cccccaaaaa atttatatta 960
   ccagttttaa ctagatactt gtaaaatata ttatctttaa aataaatatt taaattacat 1020
   ctctgctatt gtaaaacaaa cattgaattg ccataaaata aaataattaa tatacagtaa 1080
   aagggaaaaa aaacaaaaaa aatattggtt aataattcta cctcatgcta ttatatactc 1140
   cctccgtttt ttttagatgt aacattggac ttttcacatt tgccgaggca caactttgac 1200
   cgataatatc tccaattgta taaaagtaaa aattataaaa aatatatatt tggaaagtgt 1260
   ataccatgac gaatcaaaca agatcccaca tgactatatt ttttcttatg tataaataac 1320
   aattagtggt caaagtgtat tggatgaata gtgcccaaag tcaatatgtt acatctaaaa 1380
   aaaaaacgga gggagtagtg tttatttgct agacgctacc tctaaatctc aaacgttact 1440
   tgtagagttg tagtaacgtt ccacaatcgc tactcataat ctactccaac tgctacccgc 1500
   tagtacaacc actaccttac caaacactca taattttagc gtttcaatct tgtccaaccg 1560
   ttaaccgcta cctcaaatgc tagtgtcaaa taggaccaca tgtatatcga gactccatct 1620
   attcacttca tgtatgctcc ataaaaaagt aagatgtaga ttagaaagag aagagcctaa 1680
   aatagtagga ataaagaggt ggttgaacgt tgtataaaaa ctaaaaagga aatctcgtga 1740
   aaagaagaaa atataagttg acacctaata aaaaaattat gtgagattga tatgctttaa 1800
   gcatgataca tgtttatgat ctagtaacat cttttgagtt ttgaccaaaa ttatgatatt 1860
   agtacaaatt acaaatcaag aaataaagta ctcgcacaag caataattgt ttagtagaaa 1920
   acgagtggct agaaaagtta atcgcctttc taagttttna gttgnagagt tgtgggccaa 1980
   taaaaaataa ctaggtgtga tggttagatc gatattgatt attgacagtg ctactataag 2040
   tttggagggt tgggccccat cttggagatt ttctttgctg gttgggtgna tatgcttgct 2100
   cacgtatttg tggaaccata caactacaag accacacagt tcgtttgttt tcttcctttt 2160
   ttttcttaag tagtaaaaaa gagtaatcag tgttcttcat ttatctcaaa aagggatgtc 2220
   gtgtcatgac actttatttg natactttat tttaaactaa aaacttgact tacttctata 2280
   aaatagctga gtcggacact tgaacacccg tgtccgaaca tacactcaat ccgtgtctga 2340
   gtaacatagg gaataatcga tttgaattat ataacctttt tgttgattaa tttttaataa 2400
   aacaatattt caattattcc tgaataatac aattttacat cctatttact tttcgtgagt 2460
   cagaataact taaaattcgt ctaaaatatt aattcttcac cagaggagta aggacataaa 2520
   gataagggaa ggaaaaaatt gacgagttgg gaaatgtggc ttaccaaaca aaccgattat 2580
   aaacatgtca aatccattaa aagcaaatat ccaaaaaaag tttcattatt caagaataat 2640
   tgaaacattg acttattaaa atttaatcgg ctaaaggttg tattattgaa attcattatt 2700
   cattagaatc ataaaaaaca actcaattaa aaaaaatatg ggcgttgact ataatttctt 2760
   ggatcaaaac agaaaatcaa ccaaattaat aaatattttg atcaaacctg aattggccaa 2820
   atttgaccat atcagaaaac cctgataata tgataatatg attggggatg ccatgggtcg 2880
   ggattttggg cgggttcatc aacacctaga ccccagtttt agatattttt gatgtggatc 2940
   cagatctggc ttgaaacccc attgtgtcta aaatctcaaa tctataccta ttgggtctag 3000
   cgggtcctga gctaaaaatg gggttttaag atatttttct attattttat ggtctgttga 3060
   tcatttaata tttatatatt ttttctcaaa attatattca tataacgaag ttaacgctta 3120
   acgcgactac tatttaaacc tacttcagtt caaaagttta atacacgact ataaaaattc 3180
   catgacctta aagatgctta gataagtact ctcatatata catacacttt agtagattag 3240
   taattatttt taaaattcta attattaatt ctccttcatt gcatcattaa cttcaatttt 3300
   cttagaataa agagacttac ggtggtgagc atgtaaggtc ctgttctttt ggacttattt 3360
   tcagtttaat ttagtttaat tcagtttagt ttttatctac aattcagttt ctctcatttc 3420
   agatcaattt ctctcattgc aaaataacgg ggcctaagtt gtattttgca attttatata 3480
   tttattttat gaaaaaatgg atccatgggt tggatctgga gtgtatctaa gtccgacctc 3540
   attattcatg aacctagatc cgacctagga aaatttgaac cttgatttga catggacaca 3600
   tagggtctaa aaaagatccg gactcttaaa aatggggtgg gtccgacagg gtttgagtca 3660
   ggtcttagat tcattgtcat tcccaattat gaagaagctt cttgttaatc ttgtctttgt 3720
   tggacttaaa catcatatgt attgttagaa tgttagattt gagaaaattc gttgatcttg 3780
   gagtgatatc attgatcgat gtagaagagg ttacaaagtc tcagtcctaa ccgtgttaga 3840
   aattttggaa tgataaggat aatctataca caattattcc aaatgtatgc aaaactatat 3900
   actaaatatt ttctctatca tatatgttta ggattgtcta taaataacta tattttgatg 3960
   acttattatt tgggaatgta ttttatggat ttatctcgat ggtgttatat atatatatat 4020
   atatatatac tatgatctta ttcatatata attcaatatg aaattatata gaacttgact 4080
   aaccaaacct aatataccac tttactcata attgttgaac ttatttggac tttactaatt 4140
   ccttgttaaa caattatgag tttctaagta tctctagaaa gttaaaagta tagctactta 4200
   ttattgcatg caagaaactt aattccaacc ttttgttaaa aaaaaaaaaa acacttaaaa 4260
   gaaatccact ccttaatgat tatgtggaat tgtggattat ggataagaca tccacacctt 4320
   tatgactatg tggattatgg ataaaacatt cacaataact tgattattat gatgttattg 4380
   catttatttc aataaagtac tagttattag attcgggcta tgcttcgggt aagaaaaaat 4440
   tttcattatt aatataataa tttatgataa taacactaaa tacaacttag cctagtggct 4500
   aaaactttaa tatatatgct tgagttctcg tctttctcat aacgtaatag gttaaatgtt 4560
   cctaaattta acggtatact tatctagttt atacagtacg tttttctgtt ataaatccac 4620
   cacatagatt gagatttatg tgaataaaaa gaagacatag attgagagag acagtcattt 4680
   ctactttttg atgtgaatca catgggataa atataaatct ttaatcatga aaaccatagc 4740
   tgatagcacg aatgtctcat ggactgtgga catcttacct gcttctttgc cacaattttt 4800
   ttctcgactt gtggttacca atctgccaac cggggccttt atcttttata ctttttgtta 4860
   tctgtgcacc acatgctcaa taagttaatt cctcaagatg ctgatctcaa aacaaataat 4920
   aattaataat tagaaatgca agaagctgaa cacaatgatt tgagaagttt attagattta 4980
   tttcaatatt gagaactcgt ttggttgtac ttgggaagtt gatttccctt gattttcact 5040
   ttcatgaaat ttaacttcct aggaagtgag taaaattgtt tgataggcta tgggaagatg 5100
   gaattcccat aggaagtggc acttacctag gagaattagg tgcgcaactt cccccattat 5160
   gtgggaaata gaactttcta ggaagttcca cttcctatgc tctagttaaa caaacatcta 5220
   cttatacttg tacttccttg aaacttgcaa tcaggaccgt ctcataaaat taggggcctg 5280
   tgctaaatta aattataaaa attaattttt ttggtgaata ggaagagatg ggagaatcat 5340
   taatgatctt ttttggagaa taggaggaga agaaacacca aacagtaatt tctttaagtt 5400
   tgcattcaac attacacttt ataaaatttg aaagaaatac tttgtaatta gagttagctt 5460
   tttttccctt taggaggata actaatagaa tcaggctact tatattttgg ggccctgtgc 5520
   agttgctcac cctacacata gccttagccg cctttgcttg caattcctag gcaattggat 5580
   gtcaaccaaa caaggcatga atatttaatt tgtagttgac gtgatgggta tagtagtgtc 5640
   atcacatttc atttcgtatt tttgaattac aaggatcaaa tttggccttt tgagattcat 5700
   ttgagaaatt ccccttattc ctccttaagc gagcctcctt gtaaattgta actaatacaa 5760
   tttgaagcta tcaattaaaa taagaactgg agggacaaaa atgtaatata gcctcaccgt 5820
   ctaagtaatt tagactctgc ctctcctttt tagttaacac ggtttgattt tcaaatgtca 5880
   atacacactt aaaatattat tatcattaat tatcgattag taaaactaca taaaattgat 5940
   attatgaaaa tcttattgaa ataaatccaa catcgtacag aataatactc tctccgtcct 6000
   aaaataaatg aaacactttt tttttttttt tatgtattag ctttttgagg gaaactattt 6060
   catttatttc ataatgaaag ttgttttgtt tttactatat acttcctctg tttctaaata 6120
   agtgcaacac ttcttgtttt cacgtttgcc aatgcattat tttaaccgtt aatatcttca 6180
   attatcaata agtaaaaatt ataaaaagtt gatattaaaa aaattcacat tcagacgaat 6240
   ctaacaagat cccacatgac tatgtttttc ctaaaatgta aatcaccaaa gctaattaaa 6300
   gtttaaactg tgaatagtaa acactatgca aatgttgcac ttattttgaa acggaggaag 6360
   tattactcaa aaaaaaaatt agcgtgggtt gtatgaataa tacaaggtgg aagtacaaat 6420
   atattaaagt gaataaatta gatgtttttt ttattgataa agtacaaatt gcatgcttgc 6480
   aggtccactg tattacaagg gattctatca tctattttat cagtataacc ctgattcagc 6540
   tgtttggggg aacattacat ggggccatgc aatttcaacg gacctgattc actggaaata 6600
   tctcccaatc tcaatgaagc ctgatcaatg gtatgatata aatggtgttt kgacagggtc 6660
   cgctacgatc ttgccagatg gcaaaatcat gatggtctac actggtgaca ctgataaatt 6720
   tgtacaagtc caaaatttgg cataccctgc taacttatcg gatccattac ttcttgattg 6780
   ggtcaaatac ccgggtaatc ctgttttgac cccaccagag ggaatcggag ctaaagattt 6840
   tcgggatccg acgactgcat gggtggggcc cgatgggatt tggaggctta ttatcgggtc 6900
   gaaaacgggt acaacaggta tttcactagt gtacaagacc aaggatttta agacttatga 6960
   gctagagagt aacctccatg cagttccggg tacgggtatg tgggaatgtg tggattttta 7020
   cccggtgtcg atcacgggtc aaaatgggtt ggatacctcg gcttatgggt cgggtatgaa 7080
   gcacttgttg aaggctagtt tggatgataa taagcaagat cattatgcat tggggactta 7140
   tgatatgaca actcaaactt ggacacctga taacccggat atggatgtgg gtcttgggtt 7200
   gagattggat tatggtaaat attatgcgtc taagacattt tttgaccaga ataagcaaag 7260
   gaggattttg tggggttggg ttggagagac agatactgag gctgatgatt tgttgaaggg 7320
   atgggcttct ttgcaggtat aatacttcat cctttgtcct tatcatttac ataatcacgt 7380
   cattagttag ataaatacta tacacccaaa gcgtggatta tgcactaggg tgcagttgga 7440
   tagcgtggat tatgcactag gatgtagttg gatatgctca gctgaactga gcccagtcca 7500
   aatgccccgt atgtcacctt tgatataaaa attttgaatg atcttataaa gagtagcaca 7560
   cccg                                                              7564
<210> 6
   <211> 284
   <212> PRT
   <213> Beta vulgaris
<400> 6
   Pro Leu Tyr Tyr Lys Gly Phe Tyr His Leu Phe Tyr Gln Tyr Asn Pro
   1               5                   10                  15
   Asp Ser Ala Val Trp Gly Asn Ile Thr Trp Gly His Ala Ile Ser Thr
               20                  25                  30
   Asp Leu Ile His Trp Lys Tyr Leu Pro Ile Ser Met Lys Pro Asp Gln
           35                  40                  45
   Trp Tyr Asp Ile Asn Gly Val Xaa Thr Gly Ser Ala Thr Ile Leu Pro
       50                  55                  60
   Asp Gly Lys Ile Met Met Val Tyr Thr Gly Asp Thr Asp Lys Phe Val
   65                  70                  75                  80
   Gln Val Gln Asn Leu Ala Tyr Pro Ala Asn Leu Ser Asp Pro Leu Leu
                   85                  90                  95
   Leu Asp Trp Val Lys Tyr Pro Gly Asn Pro Val Leu Thr Pro Pro Glu
               100                 105                 110
   Gly Ile Gly Ala Lys Asp Phe Arg Asp Pro Thr Thr Ala Trp Val Gly
           115                 120                 125
   Pro Asp Gly Ile Trp Arg Leu Ile Ile Gly Ser Lys Thr Gly Thr Thr
       130                 135                 140
   Gly Ile Ser Leu Val Tyr Lys Thr Lys Asp Phe Lys Thr Tyr Glu Leu
   145                 150                 155                 160
   Glu Ser Asn Leu His Ala Val Pro Gly Thr Gly Met Trp Glu Cys Val
                   165                 170                 175
   Asp Phe Tyr Pro Val Ser Ile Thr Gly Gln Asn Gly Leu Asp Thr Ser
               180                 185                 190
   Ala Tyr Gly Ser Gly Met Lys His Leu Leu Lys Ala Ser Leu Asp Asp
           195                 200                 205
   Asn Lys Gln Asp His Tyr Ala Leu Gly Thr Tyr Asp Met Thr Thr Gln
       210                 215                 220
   Thr Trp Thr Pro Asp Asn Pro Asp Met Asp Val Gly Leu Gly Leu Arg
   225                 230                 235                 240
   Leu Asp Tyr Gly Lys Tyr Tyr Ala Ser Lys Thr Phe Phe Asp Gln Asn
                   245                 250                 255
   Lys Gln Arg Arg Ile Leu Trp Gly Trp Val Gly Glu Thr Asp Thr Glu
               260                 265                 270
   Ala Asp Asp Leu Leu Lys Gly Trp Ala Ser Leu Gln
           275                 280
<210> 7
   <211> 23
   <212> DNA
   <213> Beta vulgaris
<220>
   <221> misc feature
   <222> (6)
   <223> inosine
<400> 7
   atggtnsckg aycrwtggta yga 23
<210> 8
   <211> 23
   <212> DNA
   <213> Beta vulgaris
<400> 8
   tcrstrtcwg wytchccaay cca 23
<210> 9
   <211> 34
   <212> DNA
   <213> Beta vulgaris
<400> 9
   gccgaatcga gctcataaat ggtgtttgga cagg 34
<210> 10
   <211> 31
   <212> DNA
   <213> Beta vulgaris
<400> 10
   gccgttagaa gcttagccga ggtatccaac c 31